# EUROPEAN PATENT APPLICATION

(11) **EP 4 298 897 A1**
(43) Date of publication of application: **03.01.2024**
(21) Application number: 22759514.7
(22) Date of filing: 18.02.2022
(51) Int. Cl.: A01H 6/82, A24B 15/10, C12N 15/113, C12N 15/29

(54) **PLANT BODY OF GENUS NICOTIANA AND PRODUCTION METHOD THEREFOR**

(30) Priority: 26.02.2021 JP 2021031147
(71) Applicant: Japan Tobacco Inc., Tokyo 105-6927 (JP)
(72) Inventor: TAKEUCHI, Takanori, Tokyo 130-8603 (JP); UDAGAWA, Hisashi, Tokyo 130-8603 (JP); TAKAKURA, Yoshimitsu, Tokyo 130-8603 (JP); MAGOME, Hiroshi, Tokyo 130-8603 (JP); NISHIGUCHI, Ryo, Tokyo 130-8603 (JP)
(74) Representative: Hoffmann Eitle
(86) International application number: PCT/JP2022/006624
(87) International publication number: WO 2022/181478

(57) **Abstract**

Provided are a tobacco plant in which a high solavetivone content can be achieved without inoculation with a virus, and a method for producing the tobacco plant. In a tobacco plant in accordance with an aspect of the present invention, a function of an endogenous CA1 gene is suppressed.

## Description

### Technical Field

The present invention relates to a tobacco plant, and a method for producing the tobacco plant.

### Background Art

Solavetivone is one of fragrance components contained in a tobacco plant, and is known to have a citrus-like fragrance (Non-Patent Literature 1). It has also been reported that solavetivone has an inhibitory effect on sensitization and stimulation of aroma and taste of tobacco (Patent Literature 1).

It is also known that, when a virus such as a tobacco mosaic virus (TMV) is inoculated into *Nicotiana tabacum* having a resistance factor (N factor), solavetivone contained in a leaf of the tobacco plant is increased (Non-Patent Literatures 2 through 4). In the leaf inoculated with the virus, a local lesion occurs at the inoculation site, and solavetivone is produced as phytoalexin (antibacterial substance). Production of solavetivone is limited to the periphery of the lesion, and it is therefore considered that rapid programmed cell death (PCD), such as a hypersensitive response, and production of solavetivone are deeply related to each other (Non-Patent Literature 4).

### Citation List

### [Patent Literature]

[Patent Literature 1]
Japanese Patent Application Publication, Tokukaisho, No. 53-81698

### [Non-patent Literatures]

[Non-patent Literature 1]
   Fujimori, T. et. al. (1978) Neutral Volatile Components of Burley Tobacco, Beitrage zur Tabakforschung International, Vol. 9, 317-325
[Non-patent Literature 2]
   Ito, T, et. al. (1979) Solavetivone: A Stress Compound in Nicotiana tabacum Following Infection with Tobacco Mosaic Virus, Agric. Biol. Chem., 43 (2), 413-414
[Non-patent Literature 3]
   Fujimoto, Takane, et. al., 1989, Stress Compounds in Tobacco Leaves, Journal of the Chemical Society of Japan, No. 5, 889-891
[Non-patent Literature 4]
   Fujimoto, Takane, 1983, I-Kagaku To Ningen (Medicine-Science and Humans), Gakkai Shuppan Center, paper size of 788 mm × 1091 mm, 358-368
[Non-patent Literature 5]
   Zhu, X. et al. (2010) Function of endoplasmic reticulum calcium ATPase in innate immunity-mediated programmed cell death, EMBO J., 29. 1007-1018

### Summary of Invention

### Technical Problem

In a case where an attempt is made to increase an amount of solavetivone contained in a tobacco plant, a method of inoculating a virus can be considered from the phenomena disclosed in Non-Patent Literatures 2 through 4. However, there is a problem that this method is only applicable to some varieties having a resistance factor, in addition to the need for the operation of inoculating the virus.

An object of an aspect of the present invention is to provide a tobacco plant in which a high solavetivone content can be achieved without inoculation with a virus, and a method for producing the tobacco plant.

### Solution to Problem

In order to attain the object, a tobacco plant in accordance with an aspect of the present invention is a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* in which the suppression of the function is promotion by RNAi or VIGS of degradation of mRNA which has been transcribed from the at least one of the endogenous genes, as compared with a wild-type plant.

In order to attain the object, a method of producing a tobacco plant in accordance with an aspect of the present invention includes the step of introducing, in a genome of the tobacco plant, a mutation that causes suppression of a function of an endogenous gene, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant.

In order to attain the object, a tobacco leaf in accordance with an aspect of the present invention is a tobacco leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the tobacco leaf having a solavetivone content higher than that of a tobacco leaf of a wild-type tobacco plant.

In order to attain the object, a cured leaf in accordance with an aspect of the present invention is a cured leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the cured leaf having a solavetivone content higher than that of a cured leaf of a wild-type tobacco plant.

### Advantageous Effects of Invention

According to an aspect of the present invention, a tobacco plant having a high solavetivone content can be provided without undergoing a treatment such as virus inoculation.

### Brief Description of Drawings

Fig. 1 is an image obtained by taking a photograph that indicates a state of each of lines of a plant produced in Example and that is taken after 6 weeks from transplantation.
Fig. 2 is a graph showing GC-MS analysis results of respective lines of a plant produced in Example. The vertical axis represents intensity, and the horizontal axis represents time (min.)
Fig. 3 is a graph showing results of MutMap analysis.
Fig. 4 shows a phylogenetic tree based on amino acid sequences of genes of *Arabidopsis thaliana, Nicotiana tabacum* "Tsukuba 1", *Nicotiana sylvestris,* and *Nicotiana benthamiana.*
Fig. 5 shows graphs indicating a transcript amount of NtabCA1 in an NtabCA1-RNAi line (upper left), a transcript amount of NtabCA2 in an NtabCA1-RNAi line (upper right), and a transcript amount of NtabChlH in an NtabChlH-RNAi line (lower left). All values are each presented as a relative value where a transcript amount of a control line E5 is set to 1.
Fig. 6 shows an image obtained by taking a photograph indicating a state of each of leaves of NtabCA1-RNAi, NtabChlH-RNAi, and the control.
Fig. 7 is a graph indicating results of GC-MS analysis of a NtabCA1-RNAi line. The vertical axis represents intensity, and the horizontal axis represents time (min.)
Fig. 8 shows graphs indicating a transcript amount of NsCA1 in an NsCA1-RNAi line (left) and a transcript amount of NsCA2 in an NsCA1-RNAi line (right).
Fig. 9 shows an image obtained by taking a photograph indicating a state of each of individuals of wild-type *Nicotiana sylvestris,* LSC13, an NsCA1-RNAi line, and a control.
Fig. 10 shows an image obtained by taking a photograph indicating a state of each of leaves of NtabCA1 mutants.

### Description of Embodiments

### [1. Tobacco plant]

An embodiment of the present invention provides a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant.

In a tobacco plant, an endogenous gene (herein also referred to as "CA1 gene") which contains, as a coding region, a polynucleotide that encodes a polypeptide composed of an amino acid sequence represented by SEQ ID NO: 2, 4, or 6 is highly conserved with high sequence identity.

Note here that SEQ ID NO: 2 represents an amino acid sequence of a polypeptide encoded by a CA1 gene of *Nicotiana sylvestris* (herein also referred to as "NsCA1"). SEQ ID NO: 4 represents an amino acid sequence of a polypeptide encoded by a CA1 gene which is present in an S genome of *Nicotiana tabacum* (herein also referred to as "NtabCA1-S"). SEQ ID NO: 6 represents an amino acid sequence of a polypeptide encoded by a CA1 gene which is present in a T genome of *Nicotiana tabacum* (herein also referred to as "NtabCA1-T"). A polypeptide having an amino acid sequence represented by SEQ ID NO: 2 is encoded by, for example, a polynucleotide (cDNA of a CA1 gene of *Nicotiana sylvestris*) having a nucleotide sequence represented by SEQ ID NO: 1. A polypeptide having an amino acid sequence represented by SEQ ID NO: 4 is encoded by, for example, a polynucleotide (cDNA of a CA1 gene encoded by an S genome of *Nicotiana tabacum*) having a nucleotide sequence represented by SEQ ID NO: 3. A polypeptide having an amino acid sequence represented by SEQ ID NO: 6 is encoded by, for example, a polynucleotide (cDNA of a CA1 gene encoded by a T genome of *Nicotiana tabacum*) having a nucleotide sequence represented by SEQ ID NO: 5. Although not limited, herein, a polynucleotide of NsCA1 in wild-type *Nicotiana sylvestris,* a polynucleotide of NtabCA1-S in wild-type *Nicotiana tabacum,* and a polynucleotide of NtabCA1-T in wild-type *Nicotiana tabacum* can be polynucleotides that encode polypeptides of SEQ ID NOs: 2, 4, and 6, respectively. The "wild-type plant" refers to a plant in which a factor that suppresses expression of a CA1 gene has not been introduced and in which the CA1 gene is not mutated.

The CA1 gene is highly homologous to a type-IIB Ca²⁺-ATPase gene localized in the endoplasmic reticulum in *Nicotiana benthamiana.* Therefore, it is considered that a polypeptide composed of an amino acid sequence represented by SEQ ID NO: 2, 4, or 6 has Ca²⁺-ATPase activity. In Non-Patent Literature 5, it is reported that, in regard to *Nicotiana benthamiana,* in a case where transient (virus induced gene silencing (VIGS)) and constitutive (RNAi) expression-suppressed lines of type-IIB Ca²⁺-ATPase genes localized in the endoplasmic reticulum are inoculated with various viruses or elicitors, occurrence of programmed cell death is promoted as compared with a case where a control plant is inoculated with the various viruses or elicitors. However, Non-Patent Literature 5 does not disclose a phenotype in a case of no treatment in which various viruses or elicitors are not inoculated in the expression-suppressed lines of the genes. Until now, a relationship between a CA1 gene and solavetivone has not been known at all. As a result of diligent studies, the inventors of the present invention have found, for the first time, that by introducing a mutation that causes suppression of a function of a CA1 gene into a genome, it is possible to produce a tobacco plant having a high solavetivone content without carrying out a treatment such as virus inoculation, and thus completed the present invention.

The term "tobacco plant" as used herein encompasses (i) individuals as a whole (such as a mature plant, a seedling, and a seed), (ii) tissues (such as a leaf, a stem, a flower, a root, a reproductive organ, an embryo, and a part of any of these), and (iii) cured products of any of these.

The tobacco plant is not particularly limited, provided that the tobacco plant is a plant belonging to the genus *Nicotiana.* Examples of the tobacco plant encompass *Nicotiana acaulis, Nicotiana acuminata, Nicotiana acuminata var. multzjlora, Nicotiana africana, Nicotiana alata, Nicotiana amplexicaulis*, *Nicotiana arentsii, Nicotiana attenuata, Nicotiana benavidesii, Nicotiana benthamiana, Nicotiana bigelovii, Nicotiana bonariensis, Nicotiana cavicola, Nicotiana clevelandii, Nicotiana cordifolia, Nicotiana corymbosa, Nicotiana debneyi, Nicotiana excelsior, Nicotiana forgetiana, Nicotiana fragrans, Nicotiana glauca, Nicotiana glutinosa, Nicotiana goodspeedii*, *Nicotiana gossei, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon*, *Nicotiana miersii, Nicotiana noctiflora, Nicotiana nudicaulis, Nicotiana obtusifolia, Nicotiana occidentalis, Nicotiana occidentalis subsp. Hesperis, Nicotiana otophora*, *Nicotiana paniculata, Nicotiana pauczjlora*, *Nicotiana petunioides*, *Nicotiana plumbaginifolia*, *Nicotiana quadrivalvis, Nicotiana raimondii*, *Nicotiana repanda*, *Nicotiana rosulata, Nicotiana rosulata subsp. Ingulba, Nicotiana rotundifolia, Nicotiana rustica, Nicotiana setchellii, Nicotiana simulans, Nicotiana solanifolia, Nicotiana spegauinii*, *Nicotiana stocktonii, Nicotiana suaveolens, Nicotiana sylvestris, Nicotiana tabacum, Nicotiana thyrsiflora, Nicotiana tomentosa, Nicotiana tomentosiformis, Nicotiana trigonophylla*, *Nicotiana umbratica, Nicotiana undulata, Nicotiana velutina, Nicotiana wigandioides,* and hybrids of tobacco plants. Among these tobacco plants, *Nicotiana tabacum* and *Nicotiana rustica,* each of which is used as a material to produce a tobacco leaf, are particularly preferable. *Nicotiana sylvestris* can also be preferably used.

The tobacco plant in accordance with an embodiment of the present invention has a greater solavetivone content than a wild-type tobacco plant.

The term "solavetivone content" used herein refers to a content of solavetivone in a tobacco plant. The solavetivone content can be expressed by weight% relative to a cured tobacco plant. For example, in a case where a leaf is included as a tobacco plant, the solavetivone content is commonly expressed by weight% relative to a cured leaf. The term "high solavetivone content" means an increased solavetivone content as compared with a solavetivone content in a wild-type tobacco plant. The high solavetivone content or the increased solavetivone content is, for example, 2 times or more, 3 times or more, 5 times or more, and preferably 10 times or more, 20 times or more, 30 times or more, 50 times or more, and further preferably 100 times or more, of a solavetivone content in a wild-type tobacco plant.

Solavetivone can be quantitatively determined by, for example, gas chromatography-mass spectrometry (GC-MS) analysis.

The term "endogenous gene" as used herein means a gene which is originally present in a genome of a tobacco plant. That is, an endogenous gene is not an exogenous gene which is present in a plant other than a tobacco plant.

The term "sequence identity (of an amino acid sequence)" as used herein means a percentage at which a concerned (amino acid) sequence matches a reference (amino acid) sequence. Note, here, that a part of the sequence which part does not match is a part at which an amino acid residue is substituted, added, deleted, or inserted.

Note, here, that "polypeptide having a sequence identity of 95% or higher with an amino acid sequence represented by [...]", which specifies a polypeptide with use of one of amino acid sequences listed in a sequence listing, may be a polypeptide which is typically present in a tobacco plant. The terms "polypeptide" and "protein" herein have substantially the same meaning, and can therefore be used interchangeably.

Therefore, the specific polypeptide which is present in a decreased amount in the tobacco plant in accordance with an aspect of the present invention need only be a polypeptide having a sequence identity of 95% or higher with each of the amino acid sequences listed in the sequence listing. A higher sequence identity is preferable (e.g., 96%, 97%, 98%, or 99% or higher).

The phrase "suppression of a function of an endogenous gene" as used herein means a state in which a gene that is present in a genome does not fulfill its original function. Therefore, the phrase "suppression of a function of an endogenous gene" encompasses (i) "a mutation of an endogenous gene", (ii) "disruption of an endogenous gene", and (iii) "suppression of expression of an endogenous gene" by a gene (including an exogenous gene) other than the endogenous gene. A tobacco plant in accordance with the present embodiment has been achieved on the basis of the finding by the inventors of the present invention that suppression of a function of the CA1 gene increases a solavetivone content. An influence of suppression of a function of the CA1 gene on production of solavetivone has not been previously known.

As described below, *Nicotiana tabacum* is an amphidiploid and has a genome derived from its parent plant *Nicotiana sylvestris* (also referred to as an "S genome") and a genome derived from *Nicotiana tomentosiformis* (also referred to as a "T genome"). In a case where the tobacco plant is *Nicotiana tabacum,* a solavetivone content is increased by specifically suppressing a function of a CA1 gene in at least one of the S genome and the T genome. Thus, a function of a CA1 gene in one of the S genome and the T genome may be specifically suppressed, and it is preferable that functions of CA1 genes in both the S genome and the T genome are specifically suppressed.

The "mutation of an endogenous gene" means, for example, (i) a mutation of a gene (including mutation in which a polypeptide having a decreased or impaired function is produced) such that an original functional polypeptide is not produced, (ii) a mutation of a gene such that although a functional polypeptide is produced, the amount of the functional polypeptide produced is decreased, or (iii) a mutation of a gene such that although a functional polypeptide is produced, the stability of the functional polypeptide is decreased. The "disruption of an endogenous gene" means that (i) a gene which is originally present in a genome is not present in the genome or (ii) a transcript is not produced from a gene which is present in a genome. The "suppression of expression of an endogenous gene" means, for example, (1) a state in which although no change has occurred to a nucleotide of an endogenous gene, a transcriptional or translational function of the gene (from transcription into mRNA to subsequent translation into a polypeptide) is modified through another factor so that (i) the amount of the polypeptide produced is decreased or (ii) no polypeptide is produced and (II) a state in which a change has occurred to a nucleotide of the endogenous gene and degradation of mRNA by nonsense-mediated mRNA decay (described later) has occurred. The "suppression of expression of an endogenous gene" can occur as a result of, for example, degradation of mRNA which is transcribed from the endogenous gene.

As used herein, the "mutation" has the meaning ordinarily understood in the technical field to which the present application belongs, and means, for example, any change in a nucleotide present in a wild-type genome or any change in an amino acid residue present in a wild-type polypeptide (examples of these changes encompass substitution, deletion, insertion, addition, duplication, inversion, and translocation). Therefore, the "mutation of an endogenous gene" means, for example, (i) a mutation of a gene such that an original functional polypeptide is not produced, (ii) a mutation of a gene such that although a polypeptide is produced, the amount of the polypeptide produced is decreased, (iii) a mutation of a gene such that although a polypeptide is produced, the stability of the polypeptide is decreased, or (iv) a mutation of a gene such that the gene (a coding region or a full length including an untranslated region) is lost or that transcription from the gene is suppressed (e.g., a transcription-regulating region or a transcription-initiating region is deleted).

In a case where the functions are impaired by substitution, the substitution can be present in at least one of the following: a transcription-regulating sequence such as a promoter and a terminator (e.g., a sequence upstream (5' end) and a sequence downstream (3' end) with the coding region as a reference), a 5' untranslated region and a 3' untranslated region, a conserved sequence (5'GT-AG3') present at both ends of an intron, and a coding region.

For example, substitution in a nucleotide sequence which is present in a promoter sequence, a 5' untranslated region, or a 3' untranslated region of a gene and which is important in regulating expression of the gene leads to a decrease in the transcriptional activity of the gene or to a decrease in the stability of a transcript produced from the gene. Any of these decreases can lead to a reduction in transcript from the gene, and ultimately lead to a reduction in translation product. Substitution in a conserved sequence present in an intron (e.g., GT at 5' and AG at 3' in an intron) leads to splicing abnormality of mRNA. This results in abnormal mRNA in which an unnecessary intron is added or inserted. The abnormal mRNA generates an abnormal translation product or translation thereof does not terminate, due to, for example, frame shifting.

Substitution in a coding region may lead to a translation product which has an incomplete length or to a translation product which does not maintain an original function. The translation product having an incomplete length is derived from conversion, by the substitution, of a codon, which is encoding an amino acid, into a stop codon (i.e., nonsense mutation). As compared with an original translation product, the translation product which has an incomplete length is such that one or more consecutive amino acid residues including an amino acid residue at a C-terminus are deleted. The nonsense mutation occurs to any codon present upstream of an original stop codon, and is preferably present upstream of the original stop codon with one or more codons therebetween. In a case where a protein coding region of a gene has a nonsense mutation, nonsense-mediated mRNA decay (Brogna and Wen (2009), Nat. Structural Mol. Biol. 16: 107-113) may occur, and degradation of a transcript may thus occur, resulting in a decrease in transcript amount. Note that a translation product produced in this case has an incomplete length. In a case where a target gene is composed of a plurality of exons, it is preferable that at least one exon which has the nonsense mutation be present, and it is more preferable that the exon which has the nonsense mutation be not, among the plurality of exons of which the target gene is composed, an exon which is present most downstream (3' end), in order to cause the nonsense-mediated mRNA decay. The translation product which does not maintain an original function is produced due to amino acid substitution. In this case, amounts of a translation product (polypeptide) and a transcript may be equivalent to those of a translation product and a transcript in a wild-type plant. The translation product has, therein, a change in tertiary structure, a decrease in function as a functional domain, or the like. A preferred aspect of the mutation of the present invention is such amino acid substitution that leads to production of a translation product which does not maintain an original function. The amino acid substitution is preferably non-conservative substitution with a high possibility of changing the function of the translation product. For example, in Examples, a mutation had occurred in a protein-coding region (CDS, coding sequence) of a CA1 gene of mutant LSC13 having a high solavetivone content of *Nicotiana sylvestris,* and aspartic acid at position 692 in a putative amino acid sequence was substituted by asparagine. The non-conservative substitution is substitution of an amino acid by another amino acid having different properties. Examples of the non-conservative substitution encompass (i) substitution of an amino acid by another amino acid having a different electric charge or different hydrophobicity (e.g., substitution of an acidic amino acid by a neutral amino acid having no electric charge, substitution of a basic amino acid by an acidic amino acid, and substitution of a polar amino acid by a non-polar amino acid) and (ii) substitution of an amino acid by another amino acid having a side chain of a different bulk (three-dimensional size).

In a case where mutations (deletion, insertion, or the like) other than substitution occur within a promoter sequence, a 5' untranslated region, and a 3' untranslated region, a decrease may occur in transcriptional activity or stability as in the case of the substitution, so that (i) the amount of transcript may decrease and (ii) the amount of polypeptide may decrease. In addition, a mutation, other than substitution, in a conserved sequence present in an intron can lead to translation into a polypeptide having an amino acid sequence different from an original amino acid sequence, as in the case of the substitution. A mutation, other than substitution, in a coding region can lead to translation into a polypeptide having an amino acid sequence different from an original amino acid sequence, due to (i) deletion or insertion of an amino acid residue (caused by deletion or insertion of consecutive nucleotides which are multiples of 3) or (ii) frame shifting. Large deletion of an entire gene or insertion of a large fragment into the gene can cause expression itself of the gene to be lost.

In the tobacco plant, the mutation or the disruption of the at least one of the endogenous genes occurs as a result of, for example, a mutagen treatment, genetic modification, genome editing, or gene knockout. The mutagen treatment with respect to the at least one of the endogenous genes can be carried out by artificially causing a mutagen to act on the tobacco plant (as necessary, in combination with suppression of a gene repair function). Examples of the type of the mutagen encompass chemical agents such as ethyl methane sulfonate (EMS), sodium azide, ethidium bromide, and nitrous acid. Note, however, that the mutagen is not limited to these chemical agents, provided that the mutagen is a chemical agent which causes the mutation in a genomic DNA of a tobacco plant. Examples of the mutagen also encompass γ rays, heavy ion beams, X-rays, neutron rays, and UV rays. Note, however, that the mutagen is not limited to these beams and rays, provided that the mutagen is a radiation or the like which causes the mutation in a genomic DNA of a tobacco plant. The mutagen is preferably EMS. Modification of the at least one of the endogenous genes can be carried out by homologously modifying part or the whole of a target gene with use of a modifying sequence according to a publicly-known genetic modification method (gene targeting). The genome editing of the at least one of the endogenous genes can be carried out by a publicly-known technique (for example, zinc-finger nucleases (ZFN), transcription activator-like effector nucleases (TALEN), and a CRISPR/Cas9 system). The gene knockout can be carried out by, for example, insertion of a publicly-known transposon (mobile genetic factor) or T-DNA. The mutation or the disruption of the at least one of the endogenous genes is preferably not mutation or disruption that occurs as a result of spontaneous mutation.

In the case of the CRISPR/Cas9 system, genome editing can be carried out, if a guide RNA and a Cas9 protein are present in a target cell. In the case of TALEN and ZFN, the genome editing can be carried out, if a fusion protein (in which a DNA-binding domain and a nuclease are fused) is present in the target cell. Therefore, (i) the guide RNA and the Cas9 protein and (ii) the fusion protein can be each directly introduced into the target cell. Examples of a method of directly introducing any of these into the target cell encompass a PEG method, an electroporation method, and a particle bombardment method. Alternatively, it is possible that a construct in which a gene encoding a guide RNA and a Cas9 protein is connected with a desired promoter and a desired terminator is prepared in a vector, and this vector is introduced into a target cell or a target tissue using Agrobacterium or the like.

In the case of the CRISPR/Cas9 system, a sequence which is complementary to a nucleotide sequence present immediately upstream of XGG in a genome forms a base pair with a part of the guide RNA, and a double-stranded genomic DNA is cleaved by Cas9 in the nucleotide sequence.

In the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. One of the nucleotide sequences is present at one of strands of double-stranded genomic DNA, and the other of the nucleotide sequences is present at the other of the strands of the double-stranded genomic DNA. Therefore, one of the pair of DNA-binding domains binds to one of the strands, and the other of the pair of DNA-binding domains binds to the other of the strands. Each of the DNA-binding domains is composed of repeating units (modules) each of which is composed of 33 to 34 amino acid residues. The number of modules corresponds to the number of nucleotides to which the DNA-binding domain binds.

In the case of ZFN, as in the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. Each of the DNA-binding domains is composed of a plurality of zinc finger modules.

The above-described various mutations can be easily introduced into a tobacco plant by a person skilled in the art. Specifically, on the basis of these pieces of sequence information, a region which is present in a genome of any of various tobacco plants encompassed in the scope of the present invention and in which a mutation should be introduced can be determined as appropriate.

The mutation or disruption of the at least one of the endogenous genes can be determined by identifying the presence/absence of the mutation in the at least one of the endogenous genes. A method of identifying the mutation in the at least one of the endogenous genes only needs to allow the determination of the presence/absence of the mutation. Examples of the method encompass (1) a method in which a DNA sequence having the mutation is amplified by PCR or the like, and then a DNA nucleotide sequence is directly decoded with use of a commercially available sequencer or the like, (2) a method in which a difference in sequence is detected by a difference in distance of electrophoresis by a single strand conformation polymorphism (SSCP) method, (3) a method in which single nucleotide polymorphism (SNP) is detected by a CycleavePCR method, (4) a method in which the presence/absence of the mutation is identified by cleaving a mismatch site(s) with use of T7 EndonucleaseI or the like, (5) a cleaved amplified polymorphic sequence (CAPS) method in which the presence/absence of the mutation can be determined by the presence/absence of cleavage by a restriction enzyme treatment, (6) a derived CAPS (dCAPS) method in which a set of primers including a mismatch intentionally is used so that the presence/absence of the mutation can be determined by the presence/absence of cleavage by restriction enzymes, (7) a method (e.g., a PCR method in which a TaqMan probe is used) in which the presence/absence of the mutation is determined by identifying, with use of a probe which specifically hybridizes to a mutant sequence, whether or not the probe is hybridized, (8) a method (MassARRAY analysis) in which a primer that is adjacent to the mutation is used to carry out single nucleotide extension so as to detect the presence/absence of the mutation by a difference in mass between nucleotides taken in, and (9) a method in which, in a case where the mutation is deletion or insertion, the mutation is identified by a difference in mobility of electrophoresis. Alternatively, the mutation or disruption in the at least one of the endogenous genes can be determined by comparing (i) the size or the expression level of a protein which results from modification of the at least one of the endogenous genes with (ii) that of a wild-type protein. Specifically, such a comparison can be made by carrying out, for example, a Western blotting method.

Alternatively, the mutation or disruption can be analyzed by a "MutMap method". The MutMap method is a method in which bulked segregant analysis (BSA) is combined with whole genome sequencing (WGS) to identify a causative gene region of a mutant (Abe, A. et al., Nat. Biotechnol., 30(2): 174-178 (2012)). As compared with map-based cloning that is conventionally carried out, the MutMap method neither requires marker production nor requires use of a lot of individuals. This makes it possible to greatly reduce labor and time and enables more rapid gene identification.

In the MutMap method, first, a mutant line having a desired character is crossed with a parent variety (original line) used for a mutagen treatment, so that an F1 generation is obtained, and an F1 individual is further selfed, so that an F2 generation is obtained. It is considered that a character obtained by a mutation is due to a recessive mutation in many cases. Thus, a phenotype of the F1 generation is supposed to be a wild type, and a phenotype of the F2 generation is supposed to be separated into a wild type and a mutant type at a ratio of 3:1.

In the F2 generation, genetic recombination results in a random combination of a wild-type genome and a genome containing a mutation derived from a mutant, so that genomes that are uniquely combined for each individual occur. Thus, in a case where genomic DNAs derived from an F2 individual showing a mutation-type phenotype are mixed (bulked) and subjected to WGS, an expected value of an appearance frequency of reads having mutations is 0.5 for most regions of a chromosome. Meanwhile, an appearance frequency of reads having (i) a mutation causative of a phenotype shown by a mutant and (ii) a mutation in a region around the causative mutation (i) (i.e., a region linked to the causative mutation) is 1. In the MutMap method, the appearance frequency of the reads having such mutations is set as an "SNP-index", and it is determined that a causative mutant gene (or factor) is present in a region in which SNP-index = 1 is continuous.

Note that the MutMap method has been used mainly for gene analysis of rice having a relatively small genome size. The present invention has shown that the MutMap method is also applicable to gene analysis of an organism having a relatively large genome size, such as a tobacco plant.

An individual, which is generated as a result of a mutation or disruption of at least one of the endogenous genes, is herein referred to as a mutant of a tobacco plant (hereinafter simply referred to as "mutant"). Among tobacco plants, *Nicotiana tabacum* is an amphidiploid and has both a genome derived from its parent plant *Nicotiana sylvestris* (also referred to as an "S genome") and a genome derived from *Nicotiana tomentosiformis* (also referred to as a "T genome"). In *Nicotiana tabacum,* in most cases, genes indicated by an identical name are present in each of an S genome and a T genome. In the case of *Nicotiana tabacum,* the mutant can have the mutation in either the S genome or the T genome. The mutant may alternatively have the mutation in both the S genome and the T genome. Note that a single gene may have a single mutation or a plurality of mutations for causing impairment of a function. Note also that the type(s) of the mutation(s) is/are not limited. In the case of *Nicotiana tabacum,* any or all of four alleles in total, two of which are present in each of the S genome and the T genome, may have a mutation(s). In a case where mutations are present in a plurality of alleles, these mutations may be identical to or different from each other.

Suppression of expression of at least one of the endogenous genes encompass (i) suppression of transcription from the at least one of the endogenous genes to mRNA, (ii) suppression of translation from the at least one of the endogenous genes into a polypeptide through mRNA (e.g., degradation of the mRNA), and (iii) suppression of the function of the polypeptide which has been produced by the translation. The suppression of the transcription can be achieved by, for example, (i) inhibition of a transcription factor which promotes the transcription from the gene or (ii) inhibition of access of a transcription initiation factor to the gene. The suppression of the translation can be achieved with use of an antisense RNA molecule, an RNAi molecule, or a co-suppression molecule. The suppression of the function of the polypeptide can be achieved by a molecule which binds to a polypeptide that is functional and thereby inhibits the function of the polypeptide. Examples of such a molecule encompass decoy nucleic acids, ribozymes, antibodies, and inhibitory peptides.

The vector used to transform the tobacco plant for the purpose of suppression of expression of the at least one of the endogenous genes or introduction of the mutation into the at least one of the endogenous genes is not limited to any particular one, provided that a polynucleotide which is inserted in the vector can be expressed in a plant cell. Suitable examples of the vector encompass pBI, pPZP, and pSMA vectors each of which allows introduction of a target polynucleotide into a plant cell via Agrobacterium. In particular, plasmids of binary vectors (e.g., pBIG, pBIN19, pBI101, pBI121, and pPZP202) are preferable.

In a case where suppression of expression of the at least one of the endogenous genes is achieved by RNAi, a trigger sequence, which is used by RNAi to suppress expression of a target gene, is inserted into the vector. Examples of the trigger sequence encompass (i) a polynucleotide (sense RNA portion) which is (a) a part of a polynucleotide (which can have substitution of 0.1% to 1%) encoding a polypeptide having the amino acid sequence represented by SEQ ID NO: 2, 4, or 6 or a part of a polynucleotide (which can have substitution of 0.1% to 1%) having a nucleotide sequence represented by SEQ ID NO: 1, 3, or 5 and (b) represented by a nucleotide sequence of at least 21 to 30 consecutive bases (e.g., 21 or more bases, 22 or more bases, 23 or more bases, 24 or more bases, 25 or more bases, 26 or more bases, 27 or more bases, 28 or more bases, 29 or more bases, and 30 or more bases) and (ii) a polynucleotide (antisense RNA portion) which is represented by a nucleotide sequence that is complementary to the polynucleotide (i). More specifically, the nucleotide sequence of the "at least 21 to 30 consecutive bases" described above means a nucleotide sequence of 21 or more consecutive bases, 23 or more consecutive bases, 25 or more consecutive bases, 30 or more consecutive bases, 35 or more consecutive bases, 40 or more consecutive bases, 45 or more consecutive bases, 50 or more consecutive bases, 60 or more consecutive bases, 70 or more consecutive bases, 80 or more consecutive bases, 90 or more consecutive bases, or 100 or more consecutive bases.

The above suppression (of the transcription, the translation, or the function of the polypeptide) can be achieved by, for example, (i) directly introducing a molecule for achieving the suppression into a plant or (ii) introducing, into a plant, a nucleic acid molecule encoding the molecule (i.e., transformation of the plant). Note, here, that as a result of the transformation of the plant, the nucleic acid molecule is incorporated into any one or more regions of a genome of the plant.

In the tobacco plant, the suppression of the function may be a decrease in the amount of translation into the original functional polypeptide, which is a product of expression of the at least one of the endogenous genes, as compared with a wild-type plant. The translation into the polypeptide occurs based on (i) a decrease in mRNA (due to, for example, the amount of the mRNA which amount results from the instability of the mRNA itself, promotion of degradation of the mRNA, or suppression of the transcription of the mRNA) or (ii) a decrease in the amount of the translation of the mRNA (due to, for example, lack of elements (tRNA and ribosome) involved in the translation, inhibition of recruit, or functional impairment).

In the tobacco plant, the suppression of the function may be a decrease in the amount of the mRNA which has been transcribed from the at least one of the endogenous genes, as compared with a wild-type plant. The decrease in the amount of the mRNA occurs due to, for example, suppression of the transcription of the mRNA from the at least one of the endogenous genes. The suppression of the transcription can be achieved by, for example, inhibition of access of a transcription factor to the at least one of the endogenous genes, which occurs as a result of introduction of the mutation into the at least one of the endogenous genes.

The phrase "decrease in the amount of the mRNA which has been transcribed from the at least one of the endogenous genes" means that such a transcript is present at a percentage of 70% or less, 60% or less, 50% or less, 40% or less, 30% or less, 20% or less, 10% or less, 5% or less, or 1% or less with respect to the amount of a transcript produced from the at least one of the endogenous genes in a plant in which the function of the at least one of the endogenous genes is not suppressed.

In the tobacco plant, the suppression of the function may be promotion of degradation of the mRNA which has been transcribed from the at least one of the endogenous genes. The degradation of the mRNA may be caused by nonsense-mediated mRNA decay in a case where a protein coding region of a gene has a nonsense mutation. Alternatively, the degradation of the mRNA may be caused by, for example, (i) the presence of an exogenous factor leading to the degradation of the mRNA, (ii) activation of an endogenous constituent element leading to the degradation of the mRNA, or (iii) the presence of a sequence for promoting the degradation of the mRNA. In a case where the degradation of the mRNA which has been transcribed from the at least one of the endogenous genes is promoted in the tobacco plant, the mRNA in the tobacco plant is decreased. That is, in the tobacco plant, the suppression of the function may be a decrease in the amount of the mRNA which has been transcribed from the at least one of the endogenous genes, as compared with a wild-type plant.

In the tobacco plant, the mutation may be insertion, in an outside of a region in which the at least one of the endogenous genes is present, of a polynucleotide which expresses a factor that promotes the degradation of the mRNA that has been transcribed from the at least one of the endogenous genes. The factor may be an antisense RNA molecule, an RNAi molecule, or a co-suppression molecule.

In a tobacco plant in accordance with an aspect of the present invention, a function of an endogenous gene is suppressed, the endogenous gene containing, as a coding region, a polynucleotide that encodes a polypeptide composed of an amino acid sequence represented by SEQ ID NO: 2, 4, or 6.

In the tobacco plant in accordance with an aspect of the present invention, a function of an endogenous gene is suppressed, the endogenous gene containing, as a coding region, a polynucleotide that encodes a polypeptide having a sequence identity of 95% or higher with an amino acid sequence represented by SEQ ID NO: 2, 4, or 6 and having Ca²⁺-ATPase activity.

As used herein to specify an endogenous gene, "at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6" refers to any of (or any combination of) the following endogenous genes (a) through (f):
(a) an endogenous gene which contains, as a coding region, a polynucleotide that encodes a polypeptide having a sequence identity of 95% or higher with an amino acid sequence represented by SEQ ID NO: 2;
(b) an endogenous gene which contains, as a coding region, a polynucleotide that encodes a polypeptide having a sequence identity of 95% or higher with an amino acid sequence represented by SEQ ID NO: 4;
(c) an endogenous gene which contains, as a coding region, a polynucleotide that encodes a polypeptide having a sequence identity of 95% or higher with an amino acid sequence represented by SEQ ID NO: 6;
(d) a combination of (a) and (b);
(e) a combination of (a) and (c); and
(f) a combination of (b) and (c).

The "decreased amount" of the original functional polypeptide means that the polypeptide is present at a percentage of 70% or less, 60% or less, 50% or less, 40% or less, 30% or less, 20% or less, 10% or less, 5% or less, or 1% or less with respect to the amount of a wild-type polypeptide. In addition, the "decreased amount" of the original functional polypeptide includes a case where an original functional polypeptide has lost its function due to, for example, a mutation in an endogenous gene, and functional polypeptides are not present at all. The amount of the polypeptide with respect to the amount of the wild-type polypeptide can be selected, as appropriate, from the above-described values which cause a high solavetivone content in the tobacco plant.

Note that it is preferable that the above-described decrease in at least one of the amount of the mRNA and the amount of the original functional polypeptide present in the tobacco plant in accordance with an aspect of the present invention be genetically and stably inherited by a cultured cell, a callus, protoplast, a seed, and offspring each of which is obtained from the tobacco plant. Therefore, the tobacco plant in accordance with an aspect of the present invention can be an individual developed from the cultured cell, the callus, the protoplast, the seed, and the offspring, each of which has been produced through an artificial operation. Thus, these materials, from each of which the individual develops, are also encompassed in the scope of the present invention.

The tobacco plant in accordance with an aspect of the present invention can further encompass bred progeny obtained by crossing. Breeding with use of mutants has been performed in many plant species, including rice, wheat, barley, and soybean. For example, a mutant isolated from a mutant population treated with a mutagen has multiple mutations in a region other than a region of a target gene. In general, therefore, backcrossing is performed to remove an excess mutation(s). In the course of breeding, in a case where the mutant is crossed with a cultivar having an excellent character so that a character of the mutant is introduced into the cultivar, it is possible to obtain a cultivar having high additional values. A mutant has a character that is derived from a mutation. Thus, in order to proceed with backcrossing, it is necessary to select an individual having a mutation. In this case, fewer mutations that cause an intended character (high solavetivone in the present invention) reduce the number of mutations to be focused on. This reduces labor involved in backcrossing. In order to proceed with efficient backcrossing, it is necessary to carry out a method by which it is easy to determine (i) whether or not there is any mutation and (ii) whether the mutation is homozygous or heterozygous. This method can be carried out through a method of detecting a mutation (described later). In addition, in a case where marker assisted selection (MAS) is performed with use of a background marker indicative of a polymorphism between the mutant and the cultivar, it is possible to efficiently obtain, with the fewer times of crossing, a line having a high cultivar-genome-rate. A polymorphic marker can be SNP or Simple Sequence Repeat (SSR), each of which is publicly known in tobacco. If necessary, a genome sequence of tobacco to be used is analyzed so as to identify (i) a difference in nucleotide sequence and (ii) a difference in the number of repeat sequences. This allows a new polymorphic marker to be obtained and utilized.

In the tobacco plant in accordance with an aspect of the present invention, a function of an endogenous gene is suppressed, the endogenous gene containing, as a coding region, a polynucleotide that encodes a polypeptide (i) consisting of an amino acid sequence in which one or several amino acids are deleted, substituted, or added from/in/to the amino acid sequence represented by SEQ ID NO: 2, 4, or 6 and (ii) having Ca²⁺-ATPase activity. Note here that the number of amino acids that are deleted, substituted, or added from/in/to each of the amino acid sequences is, for example, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2, or 1.

In the tobacco plant in accordance with an aspect of the present invention, a function of an endogenous gene is suppressed, the endogenous gene containing, as a coding region, a polynucleotide composed of a nucleotide sequence represented by SEQ ID NO: 1, 3, or 5.

In the tobacco plant in accordance with an aspect of the present invention, a function of an endogenous gene is suppressed, the endogenous gene containing, as a coding region, a polynucleotide that encodes a polypeptide (i) hybridizing, under stringent conditions, to a polynucleotide composed of a nucleotide sequence complementary to a polynucleotide composed of a nucleotide sequence represented by SEQ ID NO: 1, 3, or 5 and (ii) having Ca²⁺-ATPase activity.

The stringent conditions indicate, in general, conditions under which (i) a double-stranded polynucleotide which is specific to a nucleotide sequence is formed but (ii) formation of a double-stranded polynucleotide which is not specific to the nucleotide sequence is markedly suppressed. In other words, the stringent conditions can be such that hybridization is carried out at a temperature falling within a range from (i) a melting temperature (Tm) of a hybrid of nucleic acids which are highly homologous to each other (e.g., a double-stranded polynucleotide which perfectly matches a probe) to (ii) a temperature 15°C lower, preferably 10°C lower, more preferably 5°C lower than the melting temperature (Tm). For example, the stringent conditions can be such that hybridization is carried out, in a common buffer solution for hybridization, at 68°C for 20 hours. In one example, hybridization is carried out, in a buffer solution (0.25 M Na2HPO4; pH 7.2; 7% SDS; 1 mM EDTA; and 1× Denhardt's solution), at 60°C to 68°C, preferably 65°C, more preferably 68°C for 16 hours to 24 hours, and then washing is carried out, in a buffer solution (20 mM Na2HPO4; pH 7.2; 1% SDS; and 1 mM EDTA), at 60°C to 68°C, preferably at 65°C, more preferably at 68°C for 15 minutes. This washing is carried out twice. In another example, prehybridization is carried out overnight at 42°C in a hybridization solution (containing 25% formamide or 50% formamide (for a more stringent condition); 4× SSC (sodium chloride/sodium citrate); 50 mM Hepes pH 7.0; 10× Denhardt's solution; and 20 µg/ml denatured salmon sperm DNA), and then hybridization is carried out by adding a labeled probe thereto and keeping a resulting solution overnight at 42°C. In washing following the hybridization, conditions of a washing solution and a temperature are approximately "1× SSC, 0.1% SDS, 37°C", approximately "0.5× SSC, 0.1% SDS, 42°C" for a more stringent condition, approximately "0.2× SSC, 0.1% SDS, 65°C" for a still more stringent condition. It can be thus expected that as the conditions of the washing following the hybridization become more stringent, DNA having higher homology to a sequence of a probe is isolated. Note, however, that the above combinations of the conditions of the SSC, the SDS, and the temperature are merely examples. A person skilled in the art can achieve stringency similar to the above by appropriately combining the above or other elements (e.g., a probe concentration, a probe length, and a time period for a hybridization reaction) that determine the stringency of hybridization. For example, a person skilled in the art can easily obtain such a gene by referring to Molecular Cloning (Sambrook, J. et al., Molecular Cloning: a Laboratory Manual 2nd ed., Cold Spring Harbor Laboratory Press, 10 Skyline Drive Plainview, NY (1989)).

### [2. Method of producing tobacco plant]

An embodiment of the present invention provides a method of producing a tobacco plant, the method including the step of introducing, in a genome of the tobacco plant, a mutation that causes suppression of a function of an endogenous gene, the endogenous gene being one of: an endogenous gene which contains, as a coding region, a polynucleotide that encodes a polypeptide having a sequence identity of 95% or higher with an amino acid sequence represented by SEQ ID NO: 2; and an endogenous gene which contains, as a coding region, a polynucleotide that encodes a polypeptide having a sequence identity of 95% or higher with an amino acid sequence represented by at least one of SEQ ID NO: 4 and SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant. Although not limited, the step of introducing the mutation in a genome of a tobacco plant can be a step of introducing the mutation into the at least one of the endogenous genes in a genome. The mutation that is introduced in the tobacco plant is described in detail in the section [1. Tobacco plant].

In the method of producing a tobacco plant, an individual exhibiting a desired phenotype can be further selected from a plant mutant population having a mutation. As an example of the selection of the individual, the following description will discuss a procedure for selecting a desired individual from a mutant population (panel) which is obtained in a case where a treatment is carried out with use of a mutagen.

A mutant of a tobacco plant which mutant the function of which is impaired due to mutations in two alleles (in the case of *Nicotiana tabacum,* a total of four alleles in both a T genome and an S genome) can be obtained by, for example, the following method. A tobacco plant is treated with use of a mutagen, as has been described above, to prepare a population (panel) of mutants each having mutations in the whole genome. Subsequently, genomic DNAs are extracted. With use of gene-specific primers in the genomes, target genes (polynucleotides) are amplified from the genomic DNAs of the panel. Thereafter, nucleotide sequences of resulting products are determined, and lines each having a homozygous mutation are then selected. For example, in the case of *Nicotiana tabacum,* first, a line (M2) having a homozygous mutation in an S genome and a line (M2) having a homozygous mutation in a T genome are obtained and then crossed to prepare F1 individuals. Subsequently, selfed progeny (F2) is developed from the F1 individuals. From the selfed progeny (F2), a line having homozygous mutations in both an S genome and a T genome is obtained.

An individual exhibiting a desired phenotype can be selected by measurement of a solavetivone content.

### [3. Others]

An embodiment of the present invention provides a method of determining a tobacco plant having a high solavetivone content. The method includes the following steps:
the step of obtaining a sample by collecting a part of a tobacco plant;
the step of detecting a mutation that causes suppression of the function of the at least one of the endogenous genes in a genome contained in the sample; and
the step of determining that a tobacco plant, in which the mutation has been detected, is a tobacco plant having a high solavetivone content.

Note here that the suppression of the function causes a high solavetivone content in a tobacco plant. That is, the determining method can be used for, for example, a method of producing a tobacco plant.

An embodiment of the present invention provides a method of breeding a tobacco plant. The breeding method includes the step of crossing tobacco plants each determined by the determining method and each having a high solavetivone content.

An embodiment of the present invention provides offspring or bred progeny, the offspring being of a tobacco plant recited above, a tobacco plant produced by a method recited above, a tobacco plant determined by a determining method recited above, or a tobacco plant produced by a breeding method recited above, the bred progeny being obtained by crossing a tobacco plant recited above, a tobacco plant produced by a method recited above, a tobacco plant determined by a determining method recited above, or a tobacco plant produced by a breeding method recited above with another tobacco plant. In the present embodiment, in the case of, for example, *Nicotiana sylvestris,* merely suppression of a function of a single CA1 gene results in an extremely high solavetivone content. This enables breeding in a mode of single-factor recessive inheritance in which mode a mutation in the CA1 gene is used as a guide. In the case of, for example, *Nicotiana tabacum,* merely suppression of a function of two CA1 genes results in an extremely high solavetivone content. This enables breeding in a mode of single-factor recessive or two-factor recessive inheritance in which mode a mutation in the CA1-S gene and/or the CA1-T gene is used as a guide.

Breeding with use of mutants has been performed in many plant species. For example, a mutant isolated from a mutant population which has been obtained by a treatment with use of a mutagen has multiple mutations in a region other than a region of a target gene. In general, therefore, backcrossing is performed to remove an excess mutation(s). In this crossing, a desired character of the mutant can be introduced into an existing cultivar by crossing the mutant with the cultivar having an excellent character. Bred progeny thus obtained can be a variety obtained by adding high values to an existing cultivar.

An embodiment of the present invention provides a tobacco leaf harvested from (i) a tobacco plant recited above, (ii) a tobacco plant produced by a method recited above, (iii) a tobacco plant determined by a determining method recited above, (iv) a tobacco plant produced by a breeding method recited above, or (v) offspring or bred progeny recited above. The tobacco leaf refers to a leaf that is harvested from a tobacco plant and used to produce a tobacco product.

A tobacco leaf in accordance with an embodiment of the present invention is a tobacco leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the tobacco leaf having a solavetivone content higher than that of a tobacco leaf of a wild-type tobacco plant.

An embodiment of the present invention provides a cured leaf obtained from the tobacco leaf. The cured leaf is obtained by curing the tobacco leaf. As a curing method, any method can be employed. The curing method can be, but is not limited to, natural curing, air curing, warm-air curing, flue curing, or the like, for example.

A cured leaf in accordance with an embodiment of the present invention is a cured leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the cured leaf having a solavetivone content higher than that of a cured leaf of a wild-type tobacco plant.

An embodiment of the present invention provides a tobacco product containing the cured leaf. The tobacco product can be in any form. The tobacco product can be, but is not limited to, shred tobaccos, cigars, paper-wrapped cigarettes, electronic tobaccos, heated tobaccos, or the like, for example. A tobacco product containing a cured leaf having a high solavetivone content can improve a flue-cured sensation (flue-cured leaf-like aroma and taste) in the aroma and taste of the tobacco product.

For details of these, a reference can be made to the above-mentioned matters concerning a tobacco plant and a method of producing the tobacco plant.

### (Recap)

With the above embodiments considered together, the present invention can be summarized as follows.
(1) A tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant.
(2) The tobacco plant recited in (1), wherein the suppression of the function is a decrease in an amount of mRNA which has been transcribed from the at least one of the endogenous genes, as compared with a wild-type plant.
(3) The tobacco plant recited in (1) or (2), wherein the suppression of the function is promotion of degradation of mRNA which has been transcribed from the at least one of the endogenous genes.
(4) The tobacco plant recited in any one of (1) through (3), wherein the mutation is introduced in the at least one of the endogenous genes.
(5) The tobacco plant recited in (4), wherein the mutation is introduced by mutagen treatment, genome editing, or gene knockout.
(6) The tobacco plant recited in (5), wherein the mutation is introduced by the mutagen treatment.
(7) The tobacco plant recited in (3), wherein the mutation is insertion, in an outside of a region in which the at least one of the endogenous genes is present, of a polynucleotide which expresses a factor that promotes the degradation of the mRNA.
(8) The tobacco plant recited in (7), wherein the factor is an antisense RNA molecule, an RNAi molecule, or a co-suppression molecule.
(9) The tobacco plant recited in any one of (1) through (8), wherein the suppression of the function is a decrease in an amount of an original functional polypeptide, as compared with a wild-type plant.
(10) The tobacco plant recited in (9), wherein the suppression of the function is a decrease in an amount of translation into an original functional polypeptide, as compared with a wild-type plant.
(11) The tobacco plant recited in any one of (1) through (10), wherein the tobacco plant belongs to *Nicotiana tabacum, Nicotiana sylvestris,* or *Nicotiana rustica.*
(12) A method of producing a tobacco plant, the method including the step of introducing, in a genome of the tobacco plant, a mutation that causes suppression of a function of an endogenous gene, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant.
(13) The method recited in (12), wherein the suppression of the function is a decrease in an amount of mRNA which has been transcribed from the at least one of the endogenous genes, as compared with a wild-type plant.
(14) The method recited in (12) or (13), wherein the suppression of the function is promotion of degradation of mRNA which has been transcribed from the at least one of the endogenous genes.
(15) The method recited in any one of (12) through (14), wherein the step of introducing the mutation includes introducing the mutation in the at least one of the endogenous genes.
(16) The method recited in (15), wherein the step of introducing the mutation is carried out by mutagen treatment, genetic modification, genome editing, or gene knockout.
(17) The tobacco plant recited in (16), wherein the mutation is introduced by the mutagen treatment.
(18) The method recited in any one of (12) through (15), wherein the step of introducing the mutation includes inserting, in an outside of a region in which the at least one of the endogenous genes is present, a polynucleotide which expresses a factor that promotes the degradation of the mRNA which has been transcribed from the at least one of the endogenous genes.
(19) The method recited in (18), wherein the factor is an antisense RNA molecule, an RNAi molecule, or a co-suppression molecule.
(20) The method recited in any one of (12) through (19), wherein the suppression of the function is a decrease in an amount of the polypeptide, as compared with a wild-type plant.
(21) The method recited in (20), wherein the suppression of the function is a decrease in an amount of translation into the polypeptide, as compared with a wild-type plant.
(22) Offspring or bred progeny, the offspring being of a tobacco plant recited in any one of (1) through (11) or a tobacco plant produced by a method recited in any one of (11) through (19), the bred progeny being obtained by crossing a tobacco plant recited in any one of (1) through (11) or a tobacco plant produced by a method recited in any one of (11) through (19) with another tobacco plant.
(23) A tobacco leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the tobacco leaf having a solavetivone content higher than that of a tobacco leaf of a wild-type tobacco plant.
(24) A cured leaf of a tobacco plant in which a mutation that causes suppression of a function of an endogenous gene is introduced in a genome, the endogenous gene being at least one of: an endogenous gene which contains, as a coding region, a polynucleotide that encodes a polypeptide having a sequence identity of 95% or higher with an amino acid sequence represented by SEQ ID NO: 2; an endogenous gene which contains, as a coding region, a polynucleotide that encodes a polypeptide having a sequence identity of 95% or higher with an amino acid sequence represented by SEQ ID NO: 4; and an endogenous gene which contains, as a coding region, a polynucleotide that encodes a polypeptide having a sequence identity of 95% or higher with an amino acid sequence represented by SEQ ID NO: 6, where the tobacco plant is not *Nicotiana benthamiana* if the suppression of the function is promotion by RNAi or VIGS of degradation of mRNA which has been transcribed from the at least one of the endogenous genes, as compared with a wild-type plant, the cured leaf having a solavetivone content higher than that of a cured leaf of a wild-type tobacco plant.
(25) A tobacco product containing a cured leaf recited in (24).

The following description will more specifically discuss an embodiment of the present invention with reference to Examples. The present invention is, of course, not limited to the Examples below and particulars can have various aspects. Further, the present invention is not limited to the embodiments, but can be altered by a skilled person in the art within the scope of the claims. An embodiment derived from a proper combination of technical means disclosed in respective different embodiments is also encompassed in the technical scope of the present invention. Moreover, all the literatures described herein are hereby incorporated by reference. Examples

### [Example 1: Isolation of Nicotiana sylvestris high solavetivone producing mutant line (LSC13 line)]

A cultivated tobacco *Nicotiana tabacum* (chromosome number: 2n = 4x = 48) is an amphidiploid derived from hybridization of (i) a tobacco wild species *Nicotiana sylvestris* (chromosome number: 2n = 2x = 24) that is a diploid and (ii) a tobacco wild species *Nicotiana tomentosiformis* (chromosome number: 2n = 2x = 24) that is also a diploid, and has two subgenomes derived from their respective ancestors (the subgenome derived from *Nicotiana sylvestris* and the subgenome derived from *Nicotiana tomentosiformis* are referred to as "S" or "S genome" and "T" or "T genome", respectively). Therefore, in *Nicotiana tabacum,* 2 copies of genes having the same function are present for many genes. In order to observe a phenotype resulted from a mutation in which a function of a target gene is lost, it is generally necessary to mutate both of two genes having functions which overlap each other.

Meanwhile, *Nicotiana sylvestris* is a diploid, and a phenotype caused by a mutation of one gene can be observed in an M2 generation following a first generation (M1 generation) in which the mutation has occurred. Thus, a *Nicotiana sylvestris* mutant library was prepared, and an attempt was made to obtain a mutant in which components of a leaf have changed.

Seeds of *Nicotiana sylvestris* were treated by immersion with 0.6% EMS for 16 hours and then washed with distilled water 8 times for 30 minutes each. The seeds thus treated were sown to grow plants of the treated generation (M1 generation). Selfed seeds (M2 generation) were obtained from each line. It was possible to obtain approximately 200 or more seeds (M2 lines) from each of 4,945 lines of EMS mutated *Nicotiana sylvestris* lines (M1 lines).

Seedlings obtained approximately 3 weeks after temporary planting in a greenhouse growing of the sown M2 lines were used. For the M2 lines of 4 to 8 individuals per line, two individuals were set as a bulk, 2 round leaf pieces were collected per individual using a metal cylinder having a diameter of 4 cm, and 4 round leaf pieces per bulk were placed in a 50-mL tube. 20 mL of methanol was added and shaken at 130 rpm for 1 hour to extract components, and then the components were subjected to GC-FID analysis. Conditions of the GC-FID analysis are shown in Table 1.

**[Table 1]**

| Item | | Condition |
|---|---|---|
| Device | | SHIMAZU GC-2010PlusAF |
| Inlet | | Splitless |
| | Inlet temperature | 250°C |
| | Sample feeding amount | 1.0 µl |
| | Purge flow rate | 3.0 ml/min |
| | Total flow rate | 49.0 ml/min |
| Column | | HP-1MS (0.25 mm x 30 m, film thickness: 1 µm) |
| | Column temperature | 40°C (2 min)→5°C/min→200°C (0 min)-1°C/min→220°C (0 min)-5°C/min→300°C (21 min) |
| | Carrier gas | He |
| | Column flow rate | 0.99 ml/min |
| Detector | | FID |
| | Detector temperature | 320°C |
| | Makeup gas | N2; 30.0 ml/min |
| | Hydrogen flow rate | 40.0 ml/min |
| | Air flow rate | 400.0 ml/min |
| Total time | | 91.0 min |

A bulk sample was selected which had a peak of a component that was quantitatively and qualitatively different from that of wild-type *Nicotiana sylvestris* (referred to as "WT") which was a control and which was not subjected to EMS treatment. A condition for selecting a quantitative change was a case in which a height of a peak (unit: µV) was 2 times or more or 1/2 or less, or a case in which the height was increased or decreased by 5,000 µV or more, as compared with WT. For the selected bulk sample, approximately 1 week after the first analysis, two pieces were sampled again from each of two separate individuals in the bulk, and then extracted with 10 mL of methanol. By carrying out GC-FID analysis again, changes in components of a leaf were reconfirmed, and individuals in which changes had occurred were selected. Among the selected individuals, for those from which selfed progeny (M3 line) was obtained, component analysis of a leaf was carried out with use of 8 individuals per line, as in the case of the M2 lines, and thus inheritance of changes in components of a leaf was determined.

As a result, 237 lines (273 individuals) of M2 lines were selected, and inheritance was confirmed in 47 lines out of 139 lines from which M3 generation seeds could be obtained. Among those, included was a line (referred to as "LSC13") in which solavetivone was markedly increased as compared with WT and a mimic lesion occurred in a leaf.

### [Example 2: Confirmation of mode of inheritance of LSC13]

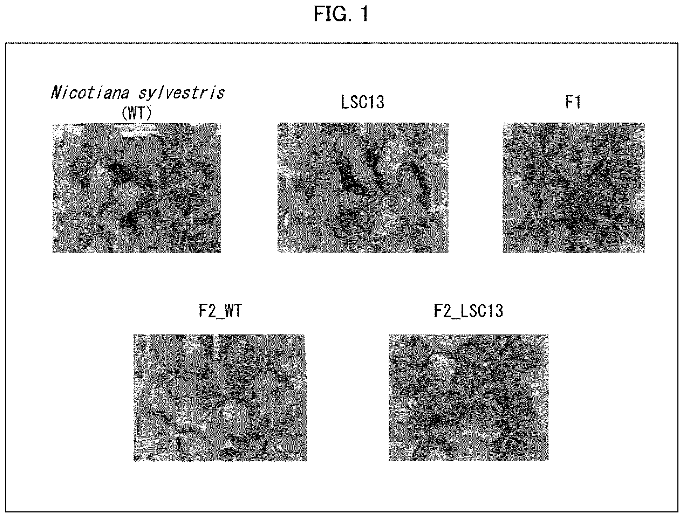

In order to investigate a mode of inheritance of LSC13, an F1 generation crossed with WT (*Nicotiana sylvestris*) was produced and further selfed, so that an F2 generation was obtained. 120 individuals of F2 individuals were grown in a greenhouse, and a phenotypic segregation ratio was determined. Five individuals of each of WT (as a control), LSC13, and F1 were grown under the same conditions. Three weeks after sowing, the individuals were temporarily planted into respective vinyl pots, and another one month later, the individuals were transplanted into respective 9-cm pots. At a time point approximately 6 weeks after transplantation, the five individuals of each of WT and F1 were not confirmed to have mimic lesions, whereas all the five individuals of LSC13 were confirmed to have mimic lesions (upper part of Fig. 1). Out of the 120 individuals of the F2 individuals, mimic lesions were identified in 28 individuals, and no mimic lesions were identified in the remaining 92 individuals (lower part of Fig. 1). In order to analyze the solavetivone content, the five individuals of each of WT, LSC13, and F1, the 28 individuals in which mimic lesions were confirmed among the F2 individuals, and 28 individuals randomly selected from the 92 individuals in which mimic lesions were not confirmed among the F2 individuals were subjected to GC-MS analysis. Leaves of the respective individuals were collected using a metal cylinder having a diameter of 4 cm, and 20 mL of methanol was added and shaken at 130 rpm for 1 hour. As an internal standard, 400 µL of eicosane (0.4 mg/mL) was added. After filtration of the extract, GC-MS analysis was carried out according to conditions indicated in Table 2.

**[Table 2]**

| Item | Condition |
|---|---|
| Device | GC-MS QP2010 Ultra |
| Feeding mode | Splitless |
| Inlet temperature | 250°C |
| Column | HP-1MS (0.25 mm x 30 m, film thickness: 1 µm) |
| Column temperature | 40°C (2 min)→5°C/min→200°C (0 min)-1°C/min→220°C (0 min)-5°C/min→300°C (21 min) |
| Carrier gas | He |
| Purge flow rate | 3.0 ml/min |
| Total flow rate | 49.0 ml/min |
| Detector | MS |
| Ion source temperature | 230°C |
| Sample feeding amount | 1.0 µl |

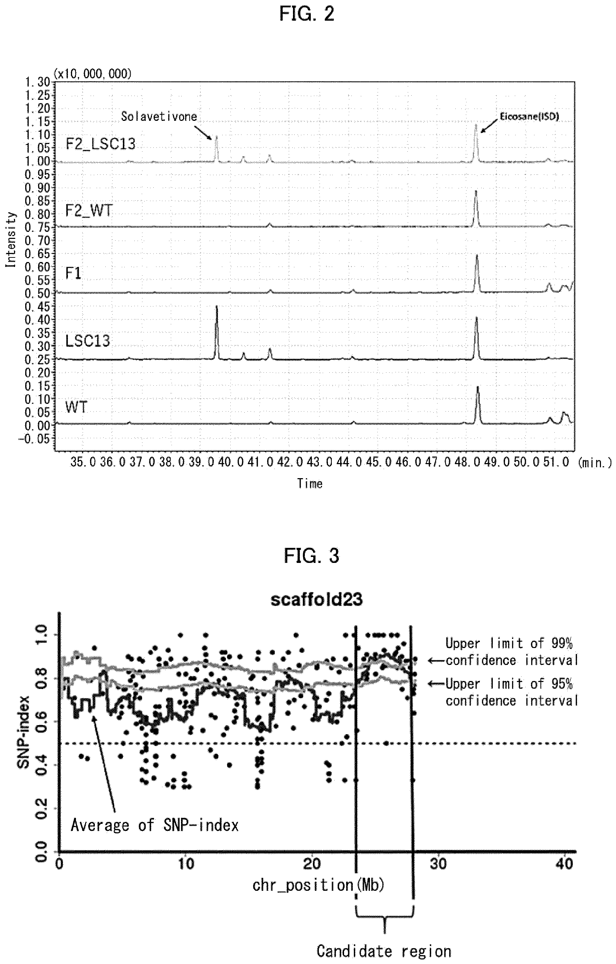

Table 3 shows results of analysis of solavetivone in each of the individuals of *Nicotiana sylvestris* (WT), LSC13, F1, and F2. A solavetivone content was presented as a ratio to the internal standard. An individual in which a mimic lesion occurred was expressed as F2_LSC13, and an individual in which a mimic lesion did not occur was expressed as F2_WT. "N.D." indicates a value below the detection limit. Solavetivone was detected in all of the individuals of LSC13 and the 28 individuals in which mimic lesions occurred among the F2 individuals. Meanwhile, solavetivone was not detected in the individuals of WT and F1, and the 28 individuals in which mimic lesions were not observed among the F2 individuals (Fig. 2, Table 3). That is, the phenotypes of the 120 individuals of F2 individuals were as follows: WT type:LSC13 type = 92:28, i.e., separated into approximately 3:1 (χ2 test, P = 0.67). This strongly suggested that an increase in solavetivone in LSC13 and a phenotype in which a mimic lesion occurs are dominated by a single recessive factor. Out of the 28 individuals of F2_LSC13, 20 individuals, excluding F2_LSC13_1, F2_LSC13_2, F2_LSC13_3, F2_LSC13_4, F2_LSC13_5, F2_LSC13_7, F2_LSC13_8 and F2_LSC13_11 (total of eight individuals), were subjected to MutMap analysis.

**[Table 3]**

| Sample | Internal standard ratio (%) | Sample | Internal standard ratio (%) | Sample | Internal standard ratio (%) |
|---|---|---|---|---|---|
| WT_1 | N.D. | F2_LSC13_1 | 0.88 | F2_WT_1 | N.D. |
| WT_2 | N.D. | F2_LSC13_2 | 2.40 | F2_WT_2 | N.D. |
| WT_3 | N.D. | F2_LSC13_3 | 3.02 | F2_WT_3 | N.D. |
| WT_4 | N.D. | F2_LSC13_4 | 3.36 | F2_WT_4 | N.D. |
| WT_5 | N.D. | F2_LSC13_5 | 8.02 | F2_WT_5 | N.D. |
| LSC13_1 | 3.5 | F2_LSC13_6 | 10.80 | F2_WT_6 | N.D. |
| LSC13_2 | 11.5 | F2_LSC13_7 | 11.21 | F2_WT_7 | N.D. |
| LSC13_3 | 68.8 | F2_LSC13_8 | 15.20 | F2_WT_8 | N.D. |
| LSC13_4 | 86.8 | F2_LSC13_9 | 17.62 | F2_WT_9 | N.D. |
| LSC13_5 | 124.7 | F2_LSC13_10 | 17.94 | F2_WT_10 | N.D. |
| F1_1 | N.D. | F2_LSC13_11 | 20.51 | F2_WT_11 | N.D. |
| F1_2 | N.D. | F2_LSC13_12 | 22.71 | F2_WT_12 | N.D. |
| F1_3 | N.D. | F2_LSC13_13 | 35.89 | F2_WT_13 | N.D. |
| F1_4 | N.D. | F2_LSC13_14 | 40.98 | F2_WT_14 | N.D. |
| F1 5 | N.D. | F2_LSC13_15 | 46.23 | F2_WT_15 | N.D. |
| | | F2_LSC13_16 | 46.70 | F2_WT_16 | N.D. |
| | | F2_LSC13_17 | 46.89 | F2_WT_17 | N.D. |
| | | F2_LSC13_18 | 51.97 | F2_WT_18 | N.D. |
| | | F2_LSC13_19 | 52.35 | F2_WT_19 | N.D. |
| | | F2_LSC13_20 | 53.04 | F2_WT_20 | N.D. |
| | | F2_LSC13_21 | 53.83 | F2_WT_21 | N.D. |
| | | F2_LSC13 22 | 57.14 | F2_WT_22 | N.D. |
| | | F2_LSC13_23 | 72.59 | F2 WT 23 | N.D. |
| | | F2 LSC13 24 | 82.52 | F2_WT_24 | N.D. |
| | | F2_LSC13_25 | 94.04 | F2_WT_25 | N.D. |
| | | F2_LSC13_26 | 112.85 | F2_WT_26 | N.D. |
| | | F2_LSC13_27 | 116.78 | F2_WT_27 | N.D. |
| | | F2_LSC13_28 | 185.41 | F2_WT_28 | N.D. |

### [Example 3: Identification of causative gene by MutMap analysis]

### 3-1. Construction of reference genome

Before carrying out MutMap analysis, a reference genome of *Nicotiana sylvestris* was constructed. A genomic DNA was extracted, according to a cetyl trimethyl ammonium bromide (CTAB) method, from a *Nicotiana sylvestris* leaf. The genomic DNA was subjected to nucleotide sequence analysis using a next-generation sequencer, so that a genome sequence was constructed. The genome sequence was used as a reference genome. The constructed reference genome consisted of 3,518 scaffolds and had N50 of 48.7 Mb.

For the purpose of prediction of a gene region in the reference genome, RNeasy Plant Mini Kit (QUIAGEN N.V.) was used to extract RNA from a total of 16 kinds of organs (n = 3) of *Nicotiana sylvestris,* which were calli (derived from a root and derived from a leaf), a germinated seed, flowers (shoot apex, bud, at 1 day after flowering, and at 4 days after flowering), leaves (at 6 weeks after sowing, at 1 week after flowering, during a maturation period (at 40 days after topping), at the 2nd day of flue curing, and at the 3rd day of air curing), roots (at 6 weeks after sowing and at 1 week after topping), a stem (at 1 week after flowering), and an axillary bud. Then the RNA thus extracted was subjected to RNA-seq by NextSeq500 (Illumina, Inc.). TruSeq (registered trademark) Stranded mRNA Library Prep (Illumina, Inc.) was used to construct an RNA-seq library.

The gene region was predicted by mapping an RNA-seq read to the reference genome and combining machine prediction with CDS prediction based on a protein sequence of a closely related plant.

### 3-2. MutMAP analysis

Among the F2 population (120 individuals) prepared by crossing LSC13 with *Nicotiana sylvestris* as described above, 28 individuals exhibited an LSC13-type phenotype. Among these 28 individuals, genomic DNAs of 20 individuals each of which had a more noticeable occurrence of mimic lesions in an upper leaf were mixed in equal amounts, so that a bulk DNA was prepared. The bulk DNA was subjected to paired-end sequence using HiSeq X (Illumina, Inc.), so that sequence data of approximately 50.48 Gb was obtained. The obtained sequence data was used to carry out MutMap analysis.

The genome sequence of *Nicotiana sylvestris* constructed above was used as the reference genome. CLC Genomic Workbench (QUIAGEN N.V.) was used for quality control of a sequence read, mapping of the sequence read to the reference genome, and extraction of a mutation.

Fig. 3 shows results of the MutMap analysis. A region in which an average of the SNP-index (score) exceeded the upper limit of a 99% confidence interval and regions which were in front and behind the above region and in which the average exceeded the upper limit of a 95% confidence interval were considered as a candidate region. As a result of the analysis, a region in which the average of the SNP-index exceeded the upper limit of the 99% confidence interval was only in a range of approximately 2.55 Mb (24.47 to 27.02 Mb) in scaffold23. A range of 4.37 Mb (23.43 to 27.80 Mb) including this region and regions which were present in front and behind the region and exceeded the 95% confidence interval were considered as a candidate region (Fig. 3), and a causative mutation was sought.

Considering the possibility that a mutation of SNP-index = 1 or a sequence error has occurred among mutations extracted in the candidate region, a mutation in which all reads except one of the reads mapped to the mutation were mutated was focused on. The following genes were considered as candidate causative genes of LSC13: genes in each of which those mutations were present in the CDS; and genes in each of which those mutations were present within 5 kb upstream of the start codon or 5 kb downstream of the stop codon in that gene and an expression level of that gene was lower in LSC13 than in WT (*Nicotiana sylvestris*)*.* The expression level of the gene was obtained by subjecting leaves of WT and LSC13 to the RNA-seq analysis.

As a result, two genes (CDS ID: nsv1s000023m05199 and nsv1s000023m05424) were extracted as candidate genes. Table 4 shows mutations in the candidate genes and expression levels of the candidate genes. In nsvls000023m05199, a mutation was present within 5 kb upstream of the gene, and the expression level was lower in LSC13 as compared with in WT (Table 4). In nsvls000023m05424, a mutation occurred in the CDS, and aspartic acid at position 692 in a putative amino acid sequence was substituted by asparagine (Table 4).

**[Table 4]**

| **Gene** | **SNP-position** | **ref** | **alt** | **No. of ref_type read** | **depth** | **SNP-index** | **WT_RPKM** | **LSC13_RPKM** | **Annotation** |
|---|---|---|---|---|---|---|---|---|---|
| nsv1s000023g0 5199 | upstream | G | A | 1 | 11 | 0.91 | 79.9 | 14.6 | Magnesium |
| | | | | | | | | | chelatase |
| | | | | | | | | | subunit H |
| nsv1s000023g0 5424 | ex3 | G | A | 0 | 8 | 1 | 38.5 | 32.4 | Calcium ATPase |

CDS sequences of both the genes were used to carry out similar sequence search (Basic Local Alignment Search Tool (BLAST), https://blast.ncbi.nlm.nih.gov/Blast.cgi) against the non-redundant (nr) database of the National Center for Biological Information (NCBI). As a result, nsvls000023m05199 showed 100% sequence homology to XM_009795182.1 (*Nicotiana sylvestris*). XM_009795182.1 was given an annotation "Magnesium (Mg)-chelatase subunit H (ChlH)" in *Nicotiana sylvestris.* Therefore, nsvls000023g05199 was referred to as NsChlH. In addition, a genomic sequence of a tobacco cultivated species *(Nicotiana tabacum)* "Tsukuba 1" was analyzed, and two homologous genes (nttvls031m00542 (referred to as NtabChlH-S, derived from the S subgenome) and nttv1s749m00829 (referred to as NtabChlH-T, derived from the T subgenome)) corresponding to NsChlH were identified. The CDS sequence of NtabChlH-S matched 100% with the sequence of XM_016613992.1 *(Nicotiana tabacum),* and the CDS sequence of NtabChlH-T matched 99.73% with the sequence of NM_001325713.1 (*Nicotiana tabacum*). Mg-chelatase is an enzyme responsible for an early reaction in synthesizing chlorophyll. Mg-chelatase is known to coordinate a magnesium ion to protoporphyrin IX and synthesize magnesium-protoporphyrin IX (Walker, J. C. et al. (1997) Biochem. J., 327(2), 321-333). Mg-chelatase is a complex protein composed of three subunits (I, D, and H) (Masuda, T. (2008) Photosyn. Res., 96(2), 121-143). It is known that, in *Arabidopsis thaliana,* a knockout or knockdown mutant of a gene encoding each of the subunits exhibits a marked decrease in chlorophyll content, and the whole plant is in white or in light green (Zhang, D., et al. (2018) Front. Plant Sci. 9, 720).

The CDS sequence (SEQ ID NO: 1) of nsvls000023m05424, which is another candidate gene, showed 100% sequence identity with XM_009802651.1 (*Nicotiana sylvestris*) and 98.3% homology with GU361620.1 (NbCA1 of *Nicotiana benthamiana*). Therefore, nsvls000023m05424 was referred to as NsCA1. A BLAST search was carried out using the CDS sequence and the amino acid sequence (SEQ ID NO: 2) of NsCA1, and gene IDs of tobacco plants hit are shown in Table 5 and Table 6. The amino acid sequence ID "XP_016480446.1" in Table 6 corresponds to the CDS sequence ID "XM_016624960.1" in Table 5. When a BLAST search was carried out on The Arabidopsis Information Resource (TAIR, https://www.arabidopsis.org/index.jsp)) for the amino acid sequence (SEQ ID NO: 2) of NsCA1, the sequence identity was highest with autoinhibited Ca²⁺-ATPase11 (ACA11: At3g57330) (amino acid sequence identity was 70%). ACA is a transmembrane protein that is localized in various intracellular membranes such as a cell membrane, an endoplasmic reticulum membrane, and a tonoplast, and uses energy by hydrolysis of ATP to transport calcium (Baxter, I., et al. (2003) Plant Physiol. 132(2) 618-628). It is known that there are 10 ACAs in *Arabidopsis thaliana* and 11 ACAs in rice (Baxter, I., et al. (2003) Plant Physiol. 132(2) 618-628). It has been reported so far that, in *Arabidopsis thaliana,* ACA11 and its paralog, ACA4, are localized in a tonoplast, and a double mutant in which functions of both of these genes are disrupted forms a mimic lesion (Boursiac Y. et al. (2010) Plant Physiol., Vol. 154, 1158-1171). It has also been reported that, in an RNAi line of NbCA1 (orthologue of nsvls000023m05424) in *Nicotiana benthamiana,* cell death around the inoculation site is promoted by inoculation with a virus or an elicitor (Non-Patent Literature 5).

**[Table 5]**

| Query | BLAST Hit | | |
|---|---|---|---|
| | ID | species | Identity (%) (query coverage) |
| CDS sequence of NsCA1 (SEQ ID NO: 1) | XM_009802651.1 | *N. sylvestris* | 100.00% (100%) |
| | XM_016639687.1 | *N. tabacum* | 99.97% (100%) |
| | GU361620.1 | *N. benthamiana* | 98.31% (100%) |
| | XM_009599438.3 | *N. tomentosiformis* | 97.93% (100%) |
| | XM_016624960.1 | *N. tabacum* | 97.93% (100%) |
| | XM_019409490.1 | *N. attenuate* | 97.51% (100%) |

**[Table 6]**

| Query | BLAST hit | | |
|---|---|---|---|
| | ID | species | Identity (%) (query coverage) |
| Amino acid sequence of NsCA1 (SEQ ID NO: 2) | XP_009800953.1 | *N. sylvestris* | 100.00% (100%) |
| | ADD91580.1 | *N. benthamiana* | 97.89% (100%) |
| | XP_009597733.1 | *N. tomentosiformis* | 97.70% (100%) |
| | XP_016480446.1 | *N. tabacum* | 97.61% (100%) |
| | XP_019265035.1 | *N. attenuata* | 97.70% (100%) |

When a BLAST search against the reference genome of *Nicotiana sylvestris,* which was newly constructed this time, was carried out using the CDS sequence of NsCA1 as a query, nsvls000146m00783 was hit with a sequence identity of 81% and was considered to be a paralog of NsCA1. Therefore, nsvls000146m00783 was referred to as NsCA2. A BLAST search was carried out in the NCBI database using the CDS sequence and the amino acid sequence of NsCA2, and gene IDs of tobacco plants hit are shown in Table 7 and Table 8. The amino acid sequence ID "XP_016457617.1" in Table 8 corresponds to the CDS sequence ID "XM_016602132.1" in Table 7.

**[Table 7]**

| Query | BLAST hit | | |
|---|---|---|---|
| | ID | species | Identity (query coverage) |
| CDS sequence of NsCA2 | XM_009784546.1 | *N. sylvestris* | 100.00% (100%) |
| | XM_019403126.1 | *N. attenuata* | 98.44% (100%) |
| | XM_033656405.1 | *N. tomentosiformis* | 96.73% (100%) |
| | XM_016602132.1 | *N. tabacum* | 96.73% (100%) |
| | XM_016623626.1 | *N. tabacum* | 99.95% (73%) |

**[Table 8]**

| Query | BLAST hit | | |
|---|---|---|---|
| | ID | species | Identity (%) (query coverage) |
| Amino acid sequence of NsCA2 | XP_009782848.1 | *N. sylvestris* | 100.00% (100%) |
| | XP_019258671.1 | *N. attenuata* | 97.55% (100%) |
| | XP_033512296.1 | *N. tomentosiformis* | 95.40% (100%) |
| | XP_016457617.1 | *N. tabacum* | 95.40% (100%) |

Next, the genomic sequence of the tobacco cultivated species *(Nicotiana tabacum)* "Tsukuba 1" was analyzed. As a result, it was found that, also in *Nicotiana tabacum,* there were two homologous genes (nttvls031m00301 (referred to as NtabCA1-S, derived from the S genome) and nttvls749m00398 (referred to as NtabCA1-T, derived from the T genome) corresponding to NsCA1 of *Nicotiana sylvestris.* A BLAST search was carried out in the NCBI database using the CDS sequence (SEQ ID NO: 3) and the amino acid sequence (SEQ ID NO: 4) of NtabCA1-S, and gene IDs of tobacco plants hit are shown in Table 9 and Table 10. Further, a BLAST search was carried out in the NCBI database using the CDS sequence (SEQ ID NO: 5) and the amino acid sequence (SEQ ID NO: 6) of NtabCA1-T, and gene IDs of tobacco plants hit are shown in Table 11 and Table 12. In Table 11, NtabCA1-T (variety: Tsukuba 1) did not perfectly match the tobacco sequence XM_016624960.1 (variety: TN90) in the DB. This was considered to be a varietal difference.

**[Table 9]**

| Query | BLAST hit | | |
|---|---|---|---|
| | ID | species | Identity (%) (query coverage) |
| CDS sequence of NtabCA1-S (SEQ ID NO: 3) | XM_016639687.1 | *N. tabacum* | 100.00% (100%) |
| | XM_009802651.1 | *N. sytvestris* | 99.97% (100%) |
| | GU361620.1 | *N. benthamiana* | 98.28% (100%) |
| | XM_009599438.3 | *N. tomentosiformis* | 97.90% (100%) |
| | XM_016624960.1 | *N. tabacum* | 97.90% (100%) |
| | XM_019409490.1 | *N. attenuata* | 97.48% (100%) |

**[Table 10]**

| Query | BLAST hit | | |
|---|---|---|---|
| | ID | species | Identity (%) (query coverage) |
| Amino acid sequence of NtabCA1-S (SEQ ID NO: 4) | XP_009800953.1 | *N. sylvestris* | 100.00% (100%) |
| | ADD91580.1 | *N. benthamiana* | 97.89% (100%) |
| | XP_009597733.1 | *N. tomentosiformis* | 97.70% (100%) |
| | XP_016480446.1 | *N. tabacum* | 97.61 % (100%) |
| | XP_019265035.1 | *N. attenuata* | 97.70% (100%) |

**[Table 11]**

| Query | BLAST hit | | |
|---|---|---|---|
| | ID | species | Identity (%) (query coverage) |
| CDS sequence of NtabCA1-T (SEQ ID NO: 5) | XM_009599438.3 | *N. tomentosiformis* | 100.00% (100%) |
| | XM_016624960.1 | *N. tabacum* | 99.94 % (100%) |
| | XM_019409490.1 | *N. attenuata* | 98.34% (100%) |
| | XM_009802651.1 | *N. sylvestris* | 97.93% (100%) |
| | XM_016639687.1 | *N. tabacum* | 97.90% (100%) |
| | GU361620.1 | *N. benthamiana* | 97.26% (100%) |

**[Table 12]**

| Query | BLAST hit | | |
|---|---|---|---|
| | ID | species | Identity (%) (query coverage) |
| Amino acid sequence of NtabCA1-T (SEQ ID NO: 6) | XP_009597733.1 | *N. tomentosiformis* | 100.00% (100%) |
| | XP_016480446.1 | *N. tabacum* | 99.90% (100%) |
| | XP_019265035.1 | *N. attenuate* | 97.99% (100%) |
| | XP_009800953.1 | *N. sylvestris* | 95.70% (100%) |
| | ADD91580.1 | *N. benthamiana* | 96.84% (100%) |

In addition, by analyzing the genomic sequence of "Tsukuba 1", two homologous genes (nttv1s264m00662 (referred to as NtabCA2-S, derived from the S genome) and nttvls227m01082 (referred to as NtabCA2-T, derived from the T genome)) corresponding to NsCA2 were identified. A BLAST search was carried out in the NCBI database using the CDS sequence and the amino acid sequence of NtabCA2-S, and gene IDs of tobacco plants hit are shown in Table 13 and Table 14. A BLAST search was carried out in the NCBI database using the CDS sequence and the amino acid sequence of NtabCA2-T, and gene IDs of tobacco plants hit are shown in Table 15 and Table 16.

**[Table 13]**

| Query | BLAST hit | | |
|---|---|---|---|
| | ID | species | Identity (%) (query coverage) |
| CDS sequence of NtabCA2-S | XM_009784546.1 | *N. sylvestris* | 99.97% (100%) |
| | XM_019403126.1 | *N. attenuata* | 98.40% (100%) |
| | XM_033656405.1 | *N. tomentosiformis* | 96.70% (100%) |
| | XM_0116602132.1 | *N. tabacum* | 96.70% (100%) |
| | XM_016623626.1 | *N. tabacum* | 100.00% (73%) |

**[Table 14]**

| Query | BLAST hit | | |
|---|---|---|---|
| | ID | species | Identity (%) (query coverage) |
| Amino acid sequence of NtabCA2-S | XP_009782848.1 | *N. sylvestris* | 99.90% (100%) |
| | XP_019258671.1 | *N. attenuata* | 97.45% (100%) |
| | XP_033512296.1 | *N. tomentosiformis* | 95.30% (100%) |
| | XP_016457617.1 | *N. tabacum* | 95.30% (100%) |

**[Table 15]**

| Query | BLAST hit | | |
|---|---|---|---|
| | ID | species | Identity (%) (query coverage) |
| CDS sequence of NtabCA2-T | XM_016602132.1 | *N. tabacum* | 100.00% (100%) |
| | XM_033656405.1 | *N. tomentosiformis* | 99.93% (100%) |
| | XM_019403126.1 | *N. attenuata* | 97.14% (100%) |
| | XM_009784546.1 | *N. sylvestris* | 96.73% (100%) |
| | XM_016623626.1 | *N. tabacum* | 96.68% (73%) |

**[Table 16]**

| Query | BLAST hit | | |
|---|---|---|---|
| | ID | species | Identity (%) (query coverage) |
| Amino acid sequence of NtabCA2-T | XP_016457617.1 | *N. tabacum* | 100.00% (100%) |
| | XP_033512296.1 | *N. tomentosiformis* | 99.80% (100%) |
| | XP_019258671.1 | *N. attenuata* | 96.22% (100%) |
| | XP_009782848.1 | *N. sylvestris* | 95.40% (100%) |

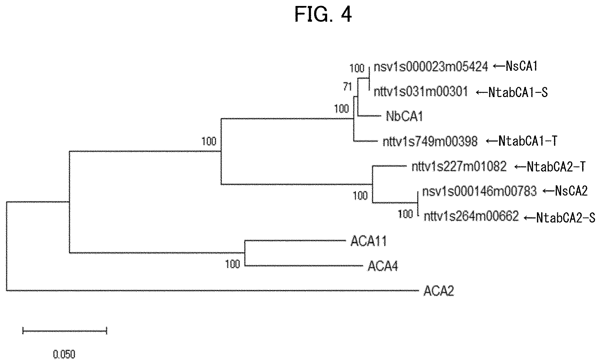

Using the amino acid sequences of CA1 and CA2 of *Nicotiana tabacum* and *Nicotiana sylvestris,* CA1 of *Nicotiana benthamiana,* and ACA11 and ACA4 of *Arabidopsis thaliana* identified as described above, a phylogenetic tree was prepared by a neighbor-joining method by MEGA X (Kumar, S., et al. (2018) Mol. Biol. Evol. 35(6) 1547-1549), and a phylogenetic relationship was determined. Fig. 4 shows a phylogenetic tree based on amino acid sequences of genes of *Arabidopsis thaliana* (ACA2, ACA4, ACA11), *Nicotiana tabacum* "Tsukuba 1" (nttv1s31m00301, nttv1s227m01082, nttv1s264m00662, nttv1s749m00398), *Nicotiana sylvestris* (nsvls000023m05424, nsv1s000146m00783), and *Nicotiana benthamiana* (NbCA1). ACA2 (At4g37640) was used as an out-group. ACA2 is known to be localized in the endoplasmic reticulum in *Arabidopsis thaliana,* and ACA11 and ACA4 are known to belong to phylogenetically different clades (Baxter, I., et al. (2003) Plant Physiol. 132(2) 618-628).

From the above information, it has been considered that NsCA1 is a potent causative gene which causes mimic lesion formation and high solavetivone property in LSC13. In order to verify this, a recombinant and a mutant in which the function of CA1 was suppressed and a recombinant and a mutant in which the function of ChlH was suppressed were produced, and their phenotypes were determined.

### [Example 4: Production of expression-suppressed line of CA1 (RNAi line)]

Expression-suppressed lines of NtabCA1 (NtabCA1-S and NtabCA1-T) and NtabChlH (NtabChlH-S and NtabChlH-T) by RNAi were produced with use of *Nicotiana tabacum* "K326". RNeasy Plant Mini Kit (QUIAGEN N.V.) was used to extract total RNA from a leaf of a maturation period of *Nicotiana sylvestris.* Then, PrimeScript (trademark) RT reagent kit with gDNA Eraser (Takara Bio Inc.) was used to synthesize cDNA. A trigger sequence was amplified using PrimeSTAR (registered trademark) Max DNA Polymerase (Takara Bio Inc.) while using the synthesized cDNAs as a template. Table 17 shows the primer sequence used to amplify the trigger of RNAi. Note that a sequence (5'-CACC-3') necessary for cloning an amplified fragment is provided at the 5' end of a forward primer (expressed as F at the end of the primer name). After sequence confirmation of the amplified fragment, the amplified fragment was cloned in pENTR (trademark)/D-TOPO (registered trademark) Vector (Thermo Fisher Scientific Inc.). Further, the trigger sequence was introduced into a binary vector pSP231 for RNAi using Gateway (trademark) LR Clonase (trademark) Enzyme mix (Thermo Fisher Scientific Inc.). The vector pSP231, which is a vector based on pHellsgates12 (see Wesley et al., Plant J., 27: 581-590 (2001)), includes a GFP expression unit. The pSP231 is a binary vector in which an RNAi sequence of a type where a pdk/cat intron is located between inverted repeat sequences of a trigger sequence is transcribed by a cauliflower mosaic virus 35SRNA gene promoter. The pSP231 vector after sequence confirmation of the trigger portion was introduced into Agrobacterium LBA4404 by electroporation. In a resulting transformed Agrobacterium, the presence of the RNAi trigger sequence was confirmed by PCR, and then the Agrobacterium was used to transform a plant. As a control, Agrobacterium into which a pSP231 vector containing no trigger sequence had been introduced was also used in transformation.

**[Table 17]**

| Target gene | Primer name | Sequence (5' → 3') | SEQ ID NO | Length of trigger |
|---|---|---|---|---|
| NtabCA1 | NtabCA1-RNAi-F | CACCTGATGCTGGTGAT CATGGTAG | 7 | 194 |
| | NtabCA1-RNAi-R | ATTTACCTGCAAGCCCTT CGAC | 8 | |
| NtabChIH | NtabChIH-RNAi-F | CACCTGTTTCCATCAAT GCCTGAG | 9 | 388 |
| | NtabChIH-RNAi-R | TCATACATACAAGGAGC CAAAG | 10 | |

A section of a leaf was infected with the Agrobacterium, and was cultured in a Linsmaier and Skoog (LS) medium containing kanamycin (50 µg/mL), so that calli were obtained. From the obtained calli, redifferentiated individuals which were kanamycin-resistant were obtained. For the redifferentiated individuals, individuals in each of which GFP fluorescence had been observed throughout a leaf were grown in a plant box and transplanted into a 12-cm pot when the individuals were sufficiently grown in the plant box, and grown in a greenhouse (24°C).

With use of RNeasy Plant Mini Kit (QUIAGEN N.V.), a total RNA was extracted from leaves of individuals approximately 3 weeks after transplantation. By reverse transcription with use of PrimeScript (trademark) RT reagent kit with gDNA Eraser (Takara Bio Inc.), from 0.5 µg of the total RNA, a genomic DNA was removed, and cDNA was synthesized. With use of 1 µL of the 3-fold diluted cDNA as a template and TaqMan (registered trademark) Fast Advanced Master Mix (Thermo Fisher Scientific Inc.), real-time PCR was carried out in a 10-µL reaction system. The expression level of a target gene was determined by a delta-delta Ct method using elfα (elongation Factor-1 α) as a reference gene. The target genes were NtabCA1 and NtabCA2 in the NtabCA1-RNAi line and NtabChlH in the NtabChlH-RNAi line. Table 18 shows sequences of primers and probes used for quantitative PCR of the genes NtabCA1, NtabCA2, NtabChlH, and elfα (internal standard gene). The primers and probes used were designed to detect both the S gene and the T gene in each of the genes.

**[Table 18]**

| Target gene | Primer/probe | Sequence (5' → 3') | SEQ ID NO |
|---|---|---|---|
| NtabCA1 | Fw primer | AATGTGTGACAGGTTTATTGATTCC | 11 |
| | Rv primer | ATATCCTTGAACGCCAAGCAAAG | 12 |
| | Probe | (FAM)ACGCAAGGCTTCATCAGCAAATT CCTTAAT(TAMRA) | 13 |
| NtabCA2 | Fw primer | AATTGTTCCTCTGACAGAAAATAGAA | 14 |
| | Rv primer | CCATCCTCAAAGTCCTTGAAGG | 15 |
| | Probe | (FAM)CACACAAAGAGTACGCAAGGCTT CGCCA(TAMRA) | 16 |
| NtabChIH | Fw primer | GCTAGACAGTGTAATCTTGACAAGG | 17 |
| | Rv primer | CGAGACTCGATCTCCATAATCTTAGA | 18 |
| | Probe | (FAM)TTTCCTACCACAAGGTCACGCTC TTTGGC(TAMRA) | 19 |
| elfa | Fw primer | CTAAGGGTGCTGCCAGCTTT | 20 |
| | Rv primer | GTCAAGCACTGGAGCATATCCA | 21 |
| | Probe | (VIC)ATCATGAACCATCCAGGACAGATT GG(TAMRA) | 22 |

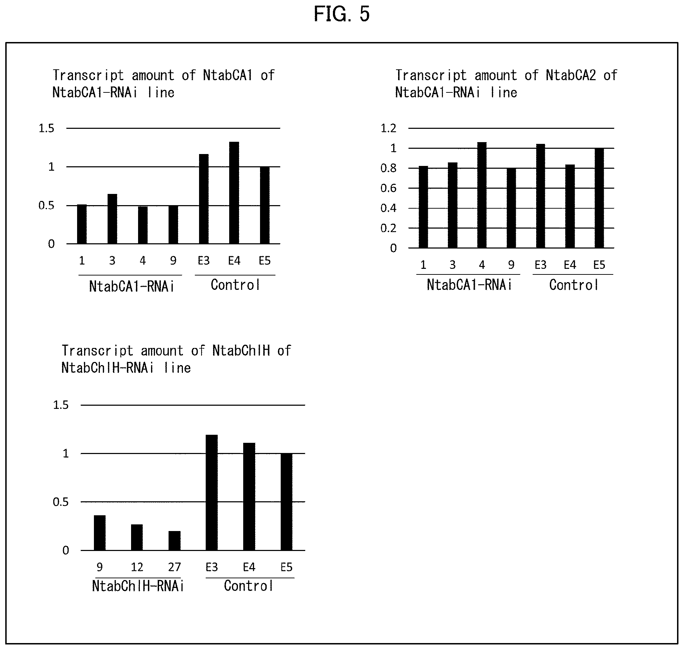

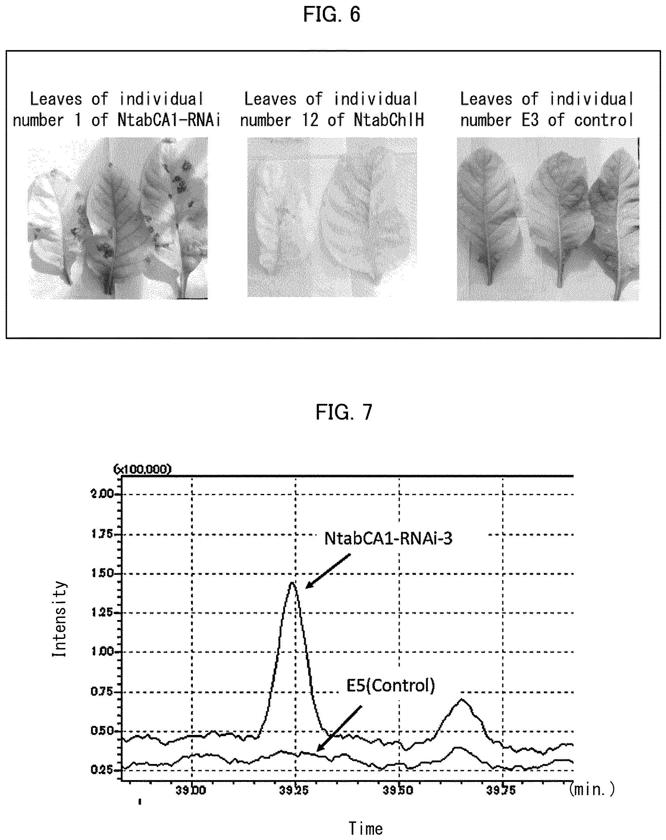

As a result, in the NtabCA1-RNAi line, the following four individuals were obtained: in the four individuals, the transcript amount of NtabCA1 was decreased by approximately half as compared with the control (i.e., a line in which the pSP231 vector containing no trigger sequence had been introduced), while the transcript amount of NtabCA2 was equivalent to that of the control (upper part of Fig. 5). In all of the expression-suppressed individuals, mimic lesions occurred approximately 1 month after transplantation, and subsequently mimic lesions continued to occur (left part of Fig. 6). In addition, three individuals were obtained in which expression of NtabChlH-RNAi was decreased as compared with the control (lower part of Fig. 5). In these three individuals, the plants as a whole including leaves showed chlorosis (middle part of Fig. 6). None of the control plants formed a mimic lesion or showed chlorosis of a plant, indicating a normal growth state (right part of Fig. 6). For four individuals of NtabCA1-RNAi, one individual of NtabChlH-RNAi, and three individuals of the control, 1 to 5 upper-middle leaves or middle leaves of each individual before flowering were collected, and then lyophilized after removal of mid-ribs. To 100 mg of the lyophilized leaves, 4 mL of methanol was added, and 80 µL (0.4 mg/mL) of eicosane was added as an internal standard. The mixture was shaken at 130 rpm for 1 hour in a 15-mL centrifugation tube to extract components. After that, the components were filtered. Note that extraction at a 3-fold concentration (300 mg of lyophilized leaves were extracted with 4 mL of methanol) was also carried out in a similar manner for the three individuals of the control. GC-MS analysis was carried out under the conditions indicated in Table 2, and the solavetivone content was measured. As a result, in the control, solavetivone was detected only in one individual extracted at the 3-fold concentration (internal standard ratio of 0.058), and solavetivone was not detected in the other individuals. Meanwhile, in the NtabCA1-RNAi, solavetivone was detected at an internal standard ratio of 0.109 to 0.377 (Fig. 7, Table 19). For the NtabChlH-RNAi individuals, solavetivone was not detected.

**[Table 19]**

| | Individual number | Internal standard ratio |
|---|---|---|
| NtabCA1 -RNAi | 1 | 0.264 |
| | 3 | 0.181 |
| | 4 | 0.109 |
| | 9 | 0.377 |
| NtabChIH-RNAi | 27 | N.D. |
| Control | E3 | N.D. |
| | E4 | N.D. |
| | E5 | N.D. |
| | E3 (3-fold concentration) | 0.058 |
| | E4 (3-fold concentration) | N.D. |
| | E5 (3-fold concentration) | N.D. |

The above results indicated that suppression of the function of CA1 caused spontaneous formation of mimic lesions and a marked increase in solavetivone content in tobacco plants.

### [Example 5: Production of expression-suppressed line of CA1 (RNAi line) in Nicotiana sylvestris]

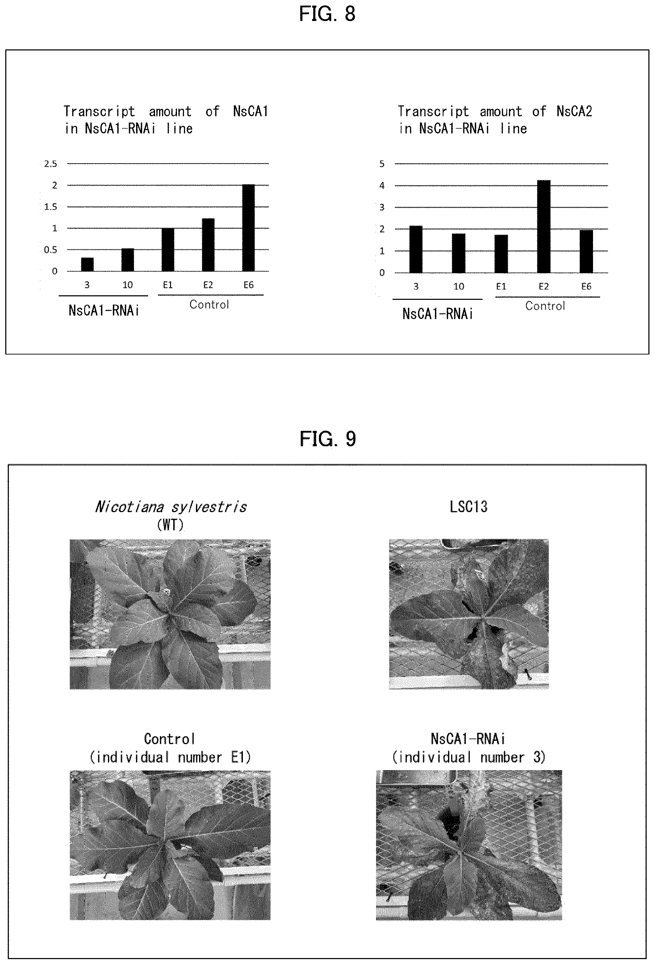

In accordance with the procedure in Example 4, *Nicotiana sylvestris* was used to produce a line (NsCA1-RNAi line) in which expression of NsCA1 was suppressed by RNAi. Confirmation of expression levels of NsCA1 and NsCA2 in redifferentiated individuals selected in accordance with presence/absence of GFP fluorescence was carried out according to the procedure of Example 4. As a result, the following two individuals were obtained: in the two individuals, the transcript amount of NsCA1 was decreased to not more than the half as compared with the control (i.e., a line in which the pSP231 vector containing no trigger sequence had been introduced), while the transcript amount of NsCA2 was equivalent to that of the control (Fig. 8). These two NsCA1-RNAi line individuals, three control individuals, five wild-type *Nicotiana sylvestris* individuals, and four LSC13 individuals were grown in a greenhouse (24°C) using 12-cm pots. In all of the NsCA1-RNAi line individuals, mimic lesions which were similar to those in LSC13 occurred approximately 1 month after transplantation to the pots (upper right and lower right of Fig. 9). None of the three individuals of the control (vector control) formed a mimic lesion, as with the wild type *Nicotiana sylvestris* (upper left and lower left of Fig. 9).

From each of the two NsCA1-RNAi line individuals, three control individuals, five wild-type *Nicotiana sylvestris* individuals, and four LSC13 individuals, 2 leaves were collected, and then lyophilized and ground after removal of mid-ribs. To 100 mg of the lyophilized and ground leaves, 4 mL of methanol was added, and 80 µL (0.4 mg/mL) of eicosane was added as an internal standard. The mixture was shaken at 130 rpm for 1 hour in a 15-mL centrifugation tube to extract components. After that, the components were filtered. GC-MS analysis was carried out under the conditions indicated in Table 2, and the solavetivone content was measured. As a result, the solavetivone content of the NsCA1-RNAi line increased 190 to 429 times that of the control (Table 20). The solavetivone content of the NsCA1-RNAi line was almost equivalent to that of LSC13. The solavetivone content of the control was almost equivalent to that of the wild-type *Nicotiana sylvestris* (Table 20).

**[Table 20]**

| | | |
|---|---|---|
| Solavetivone content of NsCA1-RNAi line | | |

| | Individual number | Internal standard ratio |
|---|---|---|
| Wild-type Nicotiana sylvestris | 1 | 0.039 |
| | 2 | 0.048 |
| | 3 | 0.048 |
| | 4 | 0.056 |
| | 5 | 0.045 |
| LSC13 | 1 | 8.156 |
| | 2 | 4.866 |
| | 3 | 0.543 |
| | 4 | 8.559 |
| NsCA 1-RNAi | 3 | 6.703 |
| | 10 | 9.179 |
| Control | 1 | 0.033 |
| | 2 | 0.021 |
| | 6 | 0.035 |

The above results indicated that suppression of the function of the NsCA1 gene in *Nicotiana sylvestris* leads to a phenotype similar to LSC13, that is, leads to formation of mimic lesions and an increase in solavetivone content. It was also suggested that amino acid substitution occurring in the NsCA1 gene of LSC13 is a mutation of a loss-of-function type.

### [Example 6: Production of mutant in which function of CA1 is suppressed in Nicotiana tabacum]

For a tobacco mutant library of 2,000 lines (Tajima et al., 2011 Annual Meeting of Phytopathological Society of Japan, p. 234, production of tobacco mutant panel) prepared by carrying out EMS treatment on seeds of the tobacco cultivated species *(Nicotiana tabacum)* "Tsukuba 1", nucleotide sequences of gene regions of the NtabCA1-S gene and the NtabCA1-T gene were analyzed, and a mutant having a mutation in any of the genes was identified. This library is constituted by a bulk DNA obtained by mixing DNAs extracted from mutant selfed progeny seeds (M2 seeds) obtained for each of 2000 individuals of EMS treated generation (M1 generation) and seedlings of 8 individuals of each of lines grown from the M2 seeds.

As a result, 2 lines having mutations in an exon region of the NtabCA1-S gene (Table 21) and 2 lines having mutations in an exon region of the NtabCA1-T gene (Table 22) were obtained. For these lines, M2 seeds were sown, and DNA was extracted from each of individuals at the time of seedling. PCR was carried out with use of this DNA as a template and primers shown in Table 23, and an individual having homozygous mutations was selected. KOD One (registered trademark) PCR Master Mix (TOYOBO CO., LTD.) was used for PCR. Note that the NtabCA1-S gene in the NtabCA1-s-1 line had an amino acid substitution identical to that of LSC13. It was confirmed that no mutations were present in the NtabCA1-T gene, the NtabCA2-S gene, and the NtabCA2-T gene in the NtabCA1-s-1 line and the NtabCA1-s-2 line, and no mutations were present in the NtabCA1-S gene, the NtabCA2-S gene, and the NtabCA2-T gene in the NtabCA1-t-1 line and the NtabCA1-t-2 line.

**[Table 21]**

| Mutant line having mutation in NtabCA1-S gene | | | | |
|---|---|---|---|---|
| Line name | Mutation type | Position in SEQ ID NO: 3 | Wild-type nucleotide | Mutant-type nucleotide |
| NtabCA1-s-1 | Missense mutation (D692N) | 2074 | G | A |
| NtabCA1-s-2 | Nonsense mutation | 521 | G | A |

**[Table 22]**

| Mutant line having mutation in NtabCA1-T gene | | | | |
|---|---|---|---|---|
| Line name | Mutation type | Position in SEQ ID NO: 5 | Wild-type nucleotide | Mutant-type nucleotide |
| NtabCA 1-t-1 | Nonsense mutation | 115 | C | T |
| NtabCA1-t-2 | Nonsense mutation | 521 | G | A |

**[Table 23]**

| Primer used to select each mutant line | | |
|---|---|---|
| Selected line | Forward primer sequence used in selection (5'→3') | Reverse primer sequence used in selection (5'→3') |
| NtabCA1-s-1 | TAAGAGACAGTGGCGTTC AAGGATATTGAA (SEQ ID NO: 23) | TAAGAGACAGTTCTTTAGC AATGGCTATAG (SEQ ID NO: 24) |
| NtabCA1-s-2 | TAAGAGACAGAGTGATGT ACCTATGAGACA (SEQ ID NO: 25) | TAAGAGACAGGTACTTTAG TTCCTGATAAA (SEQ ID NO: 26) |
| NtabCA1-t-1 | TAAGAGACAGATAAAATTT CCGGCTAACAT (SEQ ID NO: 27) | TAAGAGACAGGAGGTGAA AACATACTCTGG (SEQ ID NO: 28) |
| NtabCA1-t-2 | TAAGAGACAGTCTAGTGAT GTACCTGTAAG (SEQ ID NO: 29) | TAAGAGACAGGTACTTTG GTTCCTGATAGA (SEQ ID NO: 30) |

| | | |
|---|---|---|
| *Underlined sequences are necessary for sequencing using iSeq100 | | |

The NtabCA1-s-1 and the NtabCA1-t-1 were grown in a greenhouse and the NtabCA1-s-1 and the NtabCA1-t-1 were crossed, so that an F1 generation was obtained (NtabCA1-F1-1). The NtabCA1-s-2 and the NtabCA1-t-2 were grown in a greenhouse and the NtabCA1-s-2 and the NtabCA1-t-2 were crossed, so that an F1 generation was obtained (NtabCA1-F1-2). By growing the F1 generations in the greenhouse and selfing the F1 generations, two F2 generation lines derived from different mutant lines were obtained (NtabCA1-F2-1 and NtabCA1-F2-2).

Seeds of the two F2 generation lines were sown, DNA was extracted from seedlings, and sequences around mutations were amplified by carrying out PCR using the primers of Table 23. For the amplification products, sequences (a p7 sequence, a p5 sequence, and a bar-code sequence for each individual) used in sequencing with use of iSeq 100 (Illumina, Inc.) were further subjected to PCR to add the sequences to the amplification products. Then, the products were subjected to sequencing carried out with use of iSeq 100 (Illumina, Inc.), so that a genotype of each individual was determined.

As a result, two sets (i.e., a line derived from NtabCA1-F2-1 and a line derived from NtabCA1-F2-2) of an individual (NtabCA1-sstt) having homozygous mutations in the NtabCA1-S gene and the NtabCA1-T gene and an individual (NtabCA1-SSTT) having no mutations in both the genes were obtained.

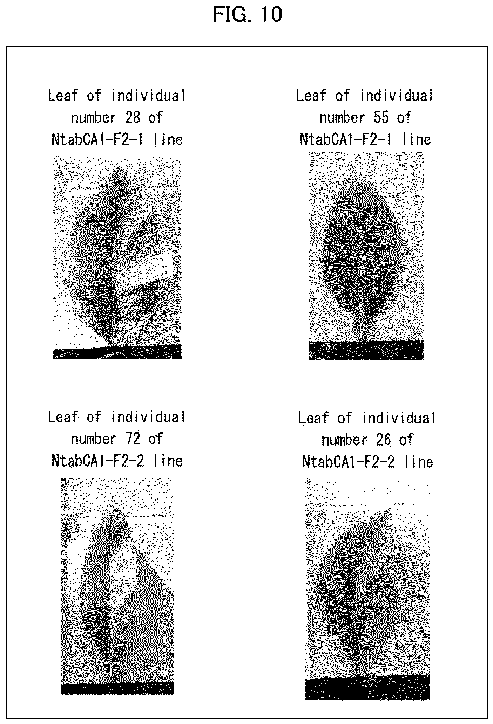

For both the line derived from NtabCA1-F2-1 and the line derived from NtabCA1-F2-2, mimic lesions occurred in the NtabCA1-sstt individuals approximately 1 month after transplantation to the pots (Tables 24 and 25, and Fig. 10). In contrast, for both the lines, mimic lesions did not occur in all of the NtabCA1-SSTT individuals (Tables 24 and 25, and Fig. 10).

Subsequently, the solavetivone content in each of the individuals of the F2 generation was investigated according to the method of Example 5 (Tables 24 and 25).

**[Table 24]**

| Presence/absence of mimic lesion and results of solavetivone analysis for each individual of NtabCA1-F2-1 line | | | |
|---|---|---|---|
| Genotype | Individual number | Mimic lesion | Solavetivone content (internal standard ratio) |
| NtabCA1-sstt | 28 | Present | 0.294 |
| | 37 | Present | 0.232 |
| | 53 | Present | 0.308 |
| | 63 | Present | 0.148 |
| | 69 | Present | 0.151 |
| NtabCA1-SSTT | 55 | None | 0.050 |
| | 80 | None | 0.017 |
| | 93 | None | 0.037 |

**[Table 25]**

| Presence/absence of mimic lesion and results of solavetivone analysis for each individual of NtabCA1-F2-Z line | | | |
|---|---|---|---|
| Genotype | Individual number | Mimic lesion | Solavetivone content (internal standard ratio) |
| NtabCA1 -sstt | 72 | Present | 0.099 |
| NtabCA1-SSTT | 26 | None | N.D. |
| | 90 | None | 0.007 |

As a result of analyzing the solavetivone content, it was found that, in the NtabCA1-sstt individuals, the solavetivone content was increased by up to 44 times as compared with that in the control NtabCA1-SSTT individuals. That is, it has found that, in *Nicotiana tabacum,* suppression of the functions of both the NtabCA1-S gene and the NtabCA1-T gene increases the solavetivone content.

No noticeable differences were found in the state of mimic lesions occurred in the line derived from NtabCA1-F2-1 and the line derived from NtabCA1-F2-2 and in the extent of the increase in solavetivone content. This suggested that amino acid substitution in the NtabCA1-S gene of the NtabCA1-F2-1 line leads to loss of the function of the NtabCA1-S gene, as with a nonsense mutation in the NtabCA1-S gene of the NtabCA1-F2-2 line.

### Industrial Applicability

The present invention provides a tobacco plant having a high solavetivone content.

## Claims

1. A tobacco plant in which a mutation that causes suppression of a function of an endogenous gene is introduced in a genome, the endogenous gene being at least one of:
an endogenous gene which contains, as a coding region, a polynucleotide that encodes a polypeptide having a sequence identity of 95% or higher with an amino acid sequence represented by SEQ ID NO: 2;
an endogenous gene which contains, as a coding region, a polynucleotide that encodes a polypeptide having a sequence identity of 95% or higher with an amino acid sequence represented by SEQ ID NO: 4; and
an endogenous gene which contains, as a coding region, a polynucleotide that encodes a polypeptide having a sequence identity of 95% or higher with an amino acid sequence represented by SEQ ID NO: 6,
where said tobacco plant is not *Nicotiana benthamiana* if the suppression of the function is promotion by RNAi or VIGS of degradation of mRNA which has been transcribed from the at least one of the endogenous genes, as compared with a wild-type plant.

2. The tobacco plant as set forth in claim 1, wherein the suppression of the function is a decrease in an amount of mRNA which has been transcribed from the at least one of the endogenous genes, as compared with a wild-type plant.

3. The tobacco plant as set forth in claim 1 or 2, wherein the suppression of the function is promotion of degradation of mRNA which has been transcribed from the at least one of the endogenous genes.

4. The tobacco plant as set forth in any one of claims 1 through 3, wherein the mutation is introduced in the at least one of the endogenous genes.

5. The tobacco plant as set forth in claim 4, wherein the mutation is introduced by mutagen treatment, genome editing, or gene knockout.

6. The tobacco plant as set forth in claim 5, wherein the mutation is introduced by the mutagen treatment.

7. The tobacco plant as set forth in claim 3, wherein the mutation is insertion, in an outside of a region in which the at least one of the endogenous genes is present, of a polynucleotide which expresses a factor that promotes the degradation of the mRNA.

8. The tobacco plant as set forth in claim 7, wherein the factor is an antisense RNA molecule, an RNAi molecule, or a co-suppression molecule.

9. The tobacco plant as set forth in any one of claims 1 through 8, wherein the suppression of the function is a decrease in an amount of an original functional polypeptide, as compared with a wild-type plant.

10. The tobacco plant as set forth in claim 9, wherein the suppression of the function is a decrease in an amount of translation into an original functional polypeptide, as compared with a wild-type plant.

11. The tobacco plant as set forth in any one of claims 1 through 10, wherein said tobacco plant belongs to *Nicotiana tabacum, Nicotiana sylvestris,* or *Nicotiana rustica.*

12. A method of producing a tobacco plant,
said method comprising the step of introducing, in a genome of the tobacco plant, a mutation that causes suppression of a function of an endogenous gene, the endogenous gene being at least one of:
an endogenous gene which contains, as a coding region, a polynucleotide that encodes a polypeptide having a sequence identity of 95% or higher with an amino acid sequence represented by SEQ ID NO: 2;
an endogenous gene which contains, as a coding region, a polynucleotide that encodes a polypeptide having a sequence identity of 95% or higher with an amino acid sequence represented by SEQ ID NO: 4; and
an endogenous gene which contains, as a coding region, a polynucleotide that encodes a polypeptide having a sequence identity of 95% or higher with an amino acid sequence represented by SEQ ID NO: 6,
where the tobacco plant is not *Nicotiana benthamiana* if the suppression of the function is promotion by RNAi or VIGS of degradation of mRNA which has been transcribed from the at least one of the endogenous genes, as compared with a wild-type plant.

13. The method as set forth in claim 12, wherein the suppression of the function is a decrease in an amount of mRNA which has been transcribed from the at least one of the endogenous genes, as compared with a wild-type plant.

14. The method as set forth in claim 12 or 13, wherein the suppression of the function is promotion of degradation of mRNA which has been transcribed from the at least one of the endogenous genes.

15. The method as set forth in any one of claims 12 through 14, wherein the step of introducing the mutation includes introducing the mutation in the at least one of the endogenous genes.

16. The method as set forth in claim 15, wherein the step of introducing the mutation is carried out by mutagen treatment, genetic modification, genome editing, or gene knockout.

17. The tobacco plant as set forth in claim 16, wherein the mutation is introduced by the mutagen treatment.

18. The method as set forth in any one of claims 12 through 15, wherein the step of introducing the mutation includes inserting, in an outside of a region in which the at least one of the endogenous genes is present, a polynucleotide which expresses a factor that promotes the degradation of the mRNA which has been transcribed from the at least one of the endogenous genes.

19. The method as set forth in claim 18, wherein the factor is an antisense RNA molecule, an RNAi molecule, or a co-suppression molecule.

20. The method as set forth in any one of claims 12 through 19, wherein the suppression of the function is a decrease in an amount of an original functional polypeptide, as compared with a wild-type plant.

21. The method as set forth in claim 20, wherein the suppression of the function is a decrease in an amount of translation into an original functional polypeptide, as compared with a wild-type plant.

22. Offspring or bred progeny,
the offspring being of a tobacco plant recited in any one of claims 1 through 11 or a tobacco plant produced by a method recited in any one of claims 11 through 19,
the bred progeny being obtained by crossing a tobacco plant recited in any one of claims 1 through 11 or a tobacco plant produced by a method recited in any one of claims 11 through 19 with another tobacco plant.

23. A tobacco leaf of a tobacco plant in which a mutation that causes suppression of a function of an endogenous gene is introduced in a genome, the endogenous gene being at least one of:
an endogenous gene which contains, as a coding region, a polynucleotide that encodes a polypeptide having a sequence identity of 95% or higher with an amino acid sequence represented by SEQ ID NO: 2;
an endogenous gene which contains, as a coding region, a polynucleotide that encodes a polypeptide having a sequence identity of 95% or higher with an amino acid sequence represented by SEQ ID NO: 4; and
an endogenous gene which contains, as a coding region, a polynucleotide that encodes a polypeptide having a sequence identity of 95% or higher with an amino acid sequence represented by SEQ ID NO: 6,
where the tobacco plant is not *Nicotiana benthamiana* if the suppression of the function is promotion by RNAi or VIGS of degradation of mRNA which has been transcribed from the at least one of the endogenous genes, as compared with a wild-type plant,
said tobacco leaf having a solavetivone content higher than that of a tobacco leaf of a wild-type tobacco plant.

24. A cured leaf of a tobacco plant in which a mutation that causes suppression of a function of an endogenous gene is introduced in a genome, the endogenous gene being at least one of:
an endogenous gene which contains, as a coding region, a polynucleotide that encodes a polypeptide having a sequence identity of 95% or higher with an amino acid sequence represented by SEQ ID NO: 2;
an endogenous gene which contains, as a coding region, a polynucleotide that encodes a polypeptide having a sequence identity of 95% or higher with an amino acid sequence represented by SEQ ID NO: 4; and
an endogenous gene which contains, as a coding region, a polynucleotide that encodes a polypeptide having a sequence identity of 95% or higher with an amino acid sequence represented by SEQ ID NO: 6,
where the tobacco plant is not *Nicotiana benthamiana* if the suppression of the function is promotion by RNAi or VIGS of degradation of mRNA which has been transcribed from the at least one of the endogenous genes, as compared with a wild-type plant,
said cured leaf having a solavetivone content higher than that of a cured leaf of a wild-type tobacco plant.

25. A tobacco product comprising a cured leaf recited in claim 24.
